(19) Europäisches Patentamt
European Patent Office
Office européen des brevets

(11) **EP 4 663 202 A1**

(12) **EUROPEAN PATENT APPLICATION**
published in accordance with Art. 153(4) EPC

(43) Date of publication:
**17.12.2025 Bulletin 2025/51**

(21) Application number: **24752931.6**

(22) Date of filing: **18.02.2024**

(51) International Patent Classification (IPC):
**A61K 39/395** (2006.01)  **C07K 16/22** (2006.01)
**A61P 35/00** (2006.01)

(52) Cooperative Patent Classification (CPC):
**A61K 39/395; A61P 35/00; C07K 16/22**

(86) International application number:
**PCT/CN2024/077354**

(87) International publication number:
**WO 2024/165077 (15.08.2024 Gazette 2024/33)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.02.2023  CN 202310101989**

(71) Applicant: **Innovent Biologics (Suzhou) Co., Ltd.
Suzhou, Jiangsu 215123 (CN)**

(72) Inventors:
• **LIU, Yi
  Suzhou, Jiangsu 215123 (CN)**

• **HE, Bing
  Suzhou, Jiangsu 215123 (CN)**
• **MA, Yidong
  Suzhou, Jiangsu 215123 (CN)**

(74) Representative: **Clark, Claudia
Redl Life Science Patents
Donau-City-Straße 11
1220 Wien (AT)**

Remarks:
The complete document including Reference
Table(s) and the Sequence Listing(s) can be
downloaded from the EPO website

(54) **PHARMACEUTICAL PREPARATION CONTAINING ANTI-VEGFA AND ANTI-VEGFC
BISPECIFIC BINDING PROTEIN**

(57)    The present application provides a pharmaceutical preparation, a use thereof, and a kit comprising the pharmaceutical preparation. The pharmaceutical preparation comprises: (1) an anti-VEGFA/VEGFC bispecific binding protein, (2) a buffering agent, (3) a stabilizer, (4) a surfactant, wherein the pharmaceutical preparation has a pH of 5.5 to 7.0. The pharmaceutical preparation shows long-term stability and exhibits anti-neovascularization effects.

**EP 4 663 202 A1**

## Description

### TECHNICAL FIELD

This application belongs to the field of pharmaceutical preparations, and in particular to a pharmaceutical preparation containing an anti-VEGFA/VEGFC bispecific binding protein.

### BACKGROUND ART

Vascular endothelial growth factor (VEGF) is a major factor regulating vascular development as well as blood and lymph vessel functions in adults when they are healthy or suffers from diseases. It is overexpressed in many human tumors. It is currently known that the VEGF family consists of five structurally related factors, including: VEGFA (vascular endothelial growth factor A; also known as VEGF-A or VEGFA165), VEGFB, VEGFC (vascular endothelial growth factor C; also known as VEGF-C), VEGFD, and placental growth factor (PLGF). Members in the VEGF family mainly occur as homodimeric polypeptides, which bind to related VEGF receptors to induce signaling and trigger corresponding biological effects. VEGFA binds to receptors VEGFR1 (also known as FLT1) and VEGFR2 (also known as KDR), and VEGFC binds to receptors VEGFR2 (also known as KDR) and VEGFR3 (also known as FLT4).

Considering the roles of VEGF-A and VEGF-C in the growth of blood and lymph vessels, researchers have proposed that VEGF-A and VEGF-C may have a synergistic promoting effect on each other in neovascularization-related diseases such as tumor growth and metastasis. Therefore, bispecific molecules capable of targeting VEGF-A and VEGF-C at the same time will help to block both vascular endothelial growth factors A and C to restrain the development of neovascularization-related diseases. Compared to the combined use of individual anti-VEGF-A molecules and individual anti-VEGF-C molecules, such bispecific molecules will also bring advantages in administration.

Bispecific antibodies are protein molecules, and as biological macromolecules, they face challenges in long-term storage. It is well known that proteins may have problems such as instability (e.g., bispecific antibodies are prone to aggregation or degradation during storage, leading to reduced safety and efficacy of these antibodies). Therefore, it is necessary to develop a corresponding pharmaceutical preparation that can enhance the stability of bispecific antibodies to ensure the in vivo therapeutic effect and clinical safety.

### SUMMARY OF THE INVENTION

In consideration of the above technical problems existing in the prior art, it is necessary to develop and provide a preparation with long-term stability for the bispecific antibodies involved in the present application, in order to facilitate their effective clinical administration.

The present application provides a pharmaceutical preparation, comprising: (1) an anti-VEGFA/VEGFC bispecific binding protein, (2) a buffering agent, (3) a stabilizer, and (4) a surfactant, wherein the pharmaceutical preparation has a pH of 5.5 to 7.0.

In some embodiments, the bispecific binding protein comprises:

(i) a first antigen binding component specifically binding to human VEGFC, wherein the first antigen binding component comprises an anti-VEGFC single-domain antibody or an anti-VEGFC VHH domain, and the single-domain antibody or the VHH domain comprises: CDR1 having an amino acid as set forth in SEQ ID NO: 1, CDR2 having an amino acid sequence as set forth in SEQ ID NO: 2, and CDR3 having an amino acid sequence as set forth in SEQ ID NO: 3; and

(ii) a second antigen binding component specifically binding to human VEGFA.

In some embodiments, the first antigen binding component (also known as anti-VEGFC component) may be a polypeptide/protein comprising the single-domain antibody of the present application or theVHH domain thereof, for example, a fusion protein (e.g., a VHH-Fc polypeptide) consisting of the VHH domain as a component and an immunoglobulin constant region (e.g. an Fc moiety, for example, a human IgG Fc region, preferably, a human IgG1 Fc region). In an embodiment, the VHH-Fc polypeptide comprises the single-domain antibody of the present application, or a VHH domain thereof, and an Fc moiety on a C-terminus of the single-domain antibody or the VHH domain thereof. In some embodiments, the human IgG1 Fc region is an Fc region comprising an amino acid sequence as set forth in SEQ ID NO: 7 or an amino acid sequence having at least 85%, 86%, 87%, 88%, 89%, 90%, 91%, 92%, 93%, 94%, 95%, 96%, 97%, 98%, 99% or more identity to the amino acid sequence as set forth in SEQ ID NO: 7. In an embodiment, the VHH-Fc polypeptide of the present application comprises an Fc moiety fused to the C-terminus of the single-domain antibody, or the VHH domain thereof, via a linkage peptide (e.g., a hinge region).

Here, the VHH domain comprised in the anti-VEGF-C single-domain antibody may be: FR1-CDR1-FR2-CDR2-FR3-CDR3-FR4. In some embodiments, the VHH domain comprises: (i) an amino acid sequence as set forth in SEQ ID NO: 4, or (ii) an amino acid sequence having at least 80%, 85%, 90%, 91%, 92%, 93%, 94%, 95%, 96%, 97%, 98%, 99% or more identity to the amino acid sequence in (i), or (iii) an amino acid sequence having at least 1 to 30, or 1 to 20, or 1 to 15, or 1 to 10, or 1 to 5 amino acid changes (e.g., substitutions, deletions and/or insertions, preferably, substitutions, and more preferably, conserved substitutions) with respect to the amino acid sequence in (i). In a preferred embodiment, the VHH domain comprises an amino acid sequence as set forth in SEQ ID NO: 4 or consists of an amino acid sequence as set forth in SEQ ID NO: 4. In an embodiment, the anti-VEGFA/VEGFC bispecific binding molecule comprises at least one, for example, two or more (preferably two), anti-VEGFC single-domain antibodies of the present application or the VHH domains thereof.

The second antigen binding component (also known as anti-VEGFA component) of the present application may be any molecule comprising a VEGFA binding domain, for example, an anti-VEGFA Trap molecule, for example, a Trap molecule comprising an extracellular domain of a VEGFA receptor VEGFR1, a Trap molecule comprising an extracellular domain of a VEGFA receptor VEGFR2, or a Trap molecule comprising extracellular domains of VEGFR1 and VEGFR2; and a fusion protein, or a chimeric polypeptide, of the VEGFA binding domain and an immunoglobulin Fc moiety.

In some embodiments, the anti-VEGFA component according to the present application is provided by an Fc chimeric polypeptide comprising the VEGFA binding domain, whereby the anti-VEGFA component is a fusion protein comprising the Fc moiety and the VEGFA binding domain.

In an embodiment, the VEGFA binding domain is a VEGFA-bindable polypeptide, which comprises or consists of: an amino acid sequence as set forth in SEQ ID NO: 6, or an amino acid sequence having at least 90%, 91%, 92%, 93%, 94%, 95%, 96%, 97%, 98%, 99% or more identity to the amino acid sequence as set forth in SEQ ID NO: 6. Preferably, the VEGFA-bindable polypeptide comprises an amino acid sequence as set forth in SEQ ID NO: 6 or consists of an amino acid sequence as set forth in SEQ ID NO: 6.

In an embodiment, the Fc moiety is a human IgG Fc region, for example, a human IgG1 Fc region, which preferably comprises or consists of: an amino acid sequence as set forth in SEQ ID NO: 7, or an amino acid sequence having at least 90%, 91%, 92%, 93%, 94%, 95%, 96%, 97%, 98%, 99% or more identity to the amino acid sequence as set forth in SEQ ID NO: 7.

In an embodiment, the anti-VEGFA component comprising the VEGFA binding domain and the Fc moiety comprises or consists of: an amino acid sequence as set forth in SEQ ID NO: 5, or an amino acid sequence having at least 90%, 91%, 92%, 93%, 94%, 95%, 96%, 97%, 98%, 99% or more identity to the amino acid sequence as set forth in SEQ ID NO: 5. In some embodiments, the second antigen binding component may be VEGF-Trap (Aflibercept, Eylea), which is a recombinant fusion protein (SEQ ID NO: 5) formed by the fusion of IgG1 Fc regions (SEQ ID NO: 7) at the C-termini of VEGF-A binding domains (SEQ ID NO: 6) from human VEGF receptors 1 and 2. The VEGF-Trap provides high-affinity binding to VEGFA in a dimeric form and blocks the activation of the VEGFR signaling pathway as induced by VEGFA.

In the bispecific binding protein of the present application, the anti-VEGFC component is linked or fused at a C-terminus or an N-terminus of the anti-VEGFA component. In some embodiments, the anti-VEGFC component may be covalently linked to the N-terminus or C-terminus (preferably, the C-terminus) of the anti-VEGFA component directly or via a linker sequence. In some embodiments, the anti-VEGFA component according to the present application binds to human VEGFA and inhibits the binding of VEGFA to its receptor(s) VEGFR1 and/or VEGFR2, as well as the signaling induced thereby.

In an embodiment where the anti-VEGFA component is an Fc fusion protein, preferably, the VEGFA binding domain may be located at the N-terminus or C-terminus of the Fc moiety; and the anti-VEGFC component is linked to an opposite end of the Fc moiety.

As understood by those skilled in the art, the components in the above fusion protein may be operably linked to allow each to perform its intended function. In an embodiment, the components of the fusion protein may be linked to each other directly or via a linker peptide or a linker. In some embodiments, the linker comprises an amino acid sequence from an immunoglobulin hinge region, or comprises a flexible amino acid sequence (e.g., a sequence consisting of glycine and serine, for example, (G4S)n or G(G4S)n, with n = 1, 2, 3, 4, 5, 6, or 7, preferably 2, 3, or 4).

In some embodiments, the bispecific binding protein of the present application may comprise a linker linking the anti-VEGFA component and the anti-VEGFC component. In an embodiment, the linker is a peptide with a length of about 6 to about 30 amino acids, for example, 10, 11, 12, 13, 14, 15, 16, 17, 18, 19, 20, 21, 22, 23, 24, 25, 26, 27, 28, 29 or 30 amino acids. Preferably, the amino acid sequence of the linker is (G4S)n or G(G4S)n, with n=1, 2, 3, 4 or 5, preferably, n=2, 3 or 4.

In some embodiments, the present application provides an anti-VEGFA/VEGFC bispecific binding protein, which comprises an anti-VEGFA component and an anti-VEGFC component, in the form of a Fc fusion protein.

In some embodiments, the anti-VEGFA/VEGFC bispecific binding protein of the present application forms a dimer through the dimerization of the Fc region. In a preferred embodiment, the VEGFA binding polypeptide of the present application and the anti-VEGFC VHH domain of the present application are fused to the N-terminus and C-terminus of the immunoglobulin Fc region, respectively, resulting in a fusion polypeptide chain that forms a dimer through the dimerization of the Fc region.

In a preferred embodiment, the bispecific binding protein of the present application comprises a first polypeptide chain and a second polypeptide chain, each of which comprises a fusion polypeptide formed by the fusion of a VEGFA-binding polypeptide and an anti-VEGFC VHH domain at the N-terminus and C-terminus of the immunoglobulin Fc region, respectively, wherein the first polypeptide chain and the second polypeptide chain may be the same or different. Preferably, the first polypeptide chain and the second polypeptide chain are the same, and the bispecific binding protein of the present application is a homodimeric protein that comprises the first polypeptide chain and the second polypeptide chain.

In some embodiments, the first polypeptide chain and the second polypeptide chain are the same and are each a VEGFA-bindable polypeptide comprising or consisting of: an amino acid sequence as set forth in SEQ ID NO: 6, or an amino acid sequence having at least 90%, 91%, 92%, 93%, 94%, 95%, 96%, 97%, 98%, 99% or more identity to the amino acid sequence as set forth in SEQ ID NO: 6. In some embodiments, the first polypeptide chain and the second polypeptide chain are the same and each comprise an anti-VEGFC VHH domain consisting of CDR1, CDR2 and CDR3 sequences as set forth in SEQ ID NOs: 1 to 3; more preferably, the VHH domain comprises an amino acid sequence as set forth in SEQ ID NO: 4, or an amino acid sequence having at least 90%, 91%, 92%, 93%, 94%, 95%, 96%, 97%, 98%, 99% or more identity to the amino acid sequence as set forth in SEQ ID NO: 4, or consists of the amino acid sequence. In another embodiment, the first polypeptide chain and the second polypeptide chain are the same and each comprise an Fc region from human IgG 1. In another embodiment, the first polypeptide chain and the second polypeptide chain are the same, and the anti-VEGFC VHH domains therein are fused at the C-terminus of the immunoglobulin Fc region via a linker.

In some embodiments, the first polypeptide chain and the second polypeptide chain are the same and each comprise an amino acid sequence as set forth in SEQ ID NO: 8.

In some embodiments, in the pharmaceutical preparation, the bispecific binding protein may have a concentration of less than 80 mg/mL and greater than 0, preferably, 20 mg/mL to 80 mg/mL, 30 mg/mL to 80 mg/mL, 40 mg/mL to 80 mg/mL, 50 mg/mL to 80 mg/mL, 20 mg/mL to 70 mg/mL, 30 mg/mL to 70 mg/mL, 40 mg/mL to 70 mg/mL, 20 mg/mL to 60 mg/mL, 30 mg/mL to 60 mg/mL, 40 mg/mL to 60 mg/mL, or 45 mg/mL to 65 mg/mL.

In some embodiments, the buffering agent is a phosphoric acid or phosphate buffering system, or a histidine buffering system.

In some preferred embodiments, the phosphoric acid or phosphate buffering system comprises phosphoric acid, sodium dihydrogen phosphate, or potassium dihydrogen phosphate.

In some preferred embodiments, the histidine buffering system comprises histidine, or histidine and L-histidine hydrochloride, preferably histidine and L-histidine hydrochloride. In some embodiments, the histidine buffering system comprises 1.0 mg/mL to 2.0 mg/mL, 1.2 mg/mL to 1.8 mg/mL, 1.3 mg/mL to 1.7 mg/mL, 1.4 mg/mL to 1.7 mg/mL, 1.4 mg/mL to 1.65 mg/mL, 1.4 mg/mL to 1.6 mg/mL, 1.45 mg/mL to 1.6 mg/mL, or 1.5 mg/mL to 1.6 mg/mL histidine. In further embodiments, the histidine buffering system comprises 0.80 mg/mL to 1.5 mg/mL, 0.80 mg/mL to 1.3 mg/mL, 0.80 mg/mL to 1.2mg/mL, 0.80 mg/mL to 1.1 mg/mL, 0.80 mg/mL to 1.0 mg/mL, 0.80-0.95 mg/mL, 0.85 mg/mL to 1.5 mg/mL, 0.85 mg/mL to 1.3 mg/mL, 0.85 mg/mL to 1.2mg/mL, 0.85 mg/mL to 1.1 mg/mL, 0.85 mg/mL to 1.0 mg/mL, or 0.85 mg/mL to 0.95 mg/mL (preferably0.90 mg/mL) histidine and 0.80 mg/mL to 1.5 mg/mL, 0.80 mg/mL to 1.3 mg/mL, 0.80 mg/mL to 1.2mg/mL, 0.80 mg/mL to 1.1 mg/mL, 0.80 mg/mL to 1.0 mg/mL, 0.80-0.95 mg/mL, 0.85 mg/mL to 1.5 mg/mL, 0.85 mg/mL to 1.3 mg/mL, 0.85 mg/mL to 1.2mg/mL, 0.85 mg/mL to 1.1 mg/mL, 0.85 mg/mL to 1.0 mg/mL, or 0.85 mg/mL to 0.95 mg/mL (preferably0.90 mg/mL) L-histidine hydrochloride.

In some embodiments, the stabilizer is a sugar, sugar alcohol or basic amino acid enabling stable presence of a protein. In some preferred embodiments, the sugar is trehalose or sucrose, for example, a sugar with a concentration of 50 mg/mL to 100 mg/mL, 60 mg/mL to 100 mg/mL, 70 mg/mL to 100 mg/mL, 80 mg/mL to 100 mg/mL, 85 mg/mL to 100 mg/mL, 50 mg/mL to 95 mg/mL, 60 mg/mL to 95 mg/mL, 70 mg/mL to 95 mg/mL, 80 mg/mL to 95 mg/mL, 85 mg/mL to 95 mg/mL, 50 mg/mL to 90 mg/mL, 60 mg/mL to 90 mg/mL, 70 mg/mL to 90 mg/mL, 80 mg/mL to 90 mg/mL, or 85 mg/mL to 90 mg/mL (e.g., trehalose with a concentration of 80 mg/mL to 100 mg/mL, 85 mg/mL to 100 mg/mL, 85 mg/mL to 95 mg/mL, 80 mg/mL to 90 mg/mL, or 85 mg/mL to 90 mg/mL). In some preferred embodiments, the sugar alcohol is sorbitol or mannitol, for example, a sugar alcohol with a concentration of 30 mg/mL to 80 mg/mL, 35 mg/mL to 70 mg/mL, 40 mg/mL to 60 mg/mL, 45 mg/mL to 55 mg/mL, or 50 mg/mL to 55 mg/mL (e.g., sorbitol with a concentration of 40 mg/mL to 60 mg/mL, 45 mg/mL to 55 mg/mL, or 50 mg/mL to 55 mg/mL). In some preferred embodiments, the basic amino acid is arginine or lysine, for example, a basic amino acid with a concentration of 5 mg/mL to 30 mg/mL, 8 mg/mL to 25 mg/mL, 9 mg/mL to 25 mg/mL, 10 mg/mL to 25 mg/mL, 11 mg/mL to 25 mg/mL, 12 mg/mL to 25 mg/mL, 8 mg/mL to 20 mg/mL, 9 mg/mL to 20 mg/mL, 10 mg/mL to 20 mg/mL, 11 mg/mL to 20 mg/mL, 12 mg/mL to 20 mg/mL, 10 mg/mL to 15 mg/mL, 11 mg/mL to 15 mg/mL, 12 mg/mL to 15 mg/mL, or 13 mg/mL to 15 mg/mL (e.g., arginine with a concentration of 10 mg/mL to 15 mg/mL, 11 mg/mL to 15 mg/mL, 12 mg/mL to 15 mg/mL, or 13 mg/mL to 15 mg/mL).

In some embodiments, the surfactant is a non-ionic surfactant, preferably polysorbate (e.g., polysorbate 20 or polysorbate 80), preferably polysorbate with a concentration of 0.1 mg/mL to 0.5 mg/mL, 0.2 mg/mL to 0.5 mg/mL, 0.25 mg/mL to 0.5 mg/mL, 0.2 mg/mL to 0.45 mg/mL, 0.25 mg/mL to 0.45 mg/mL, 0.2 mg/mL to 0.4 mg/mL, or 0.25 mg/mL to 0.4 mg/mL, preferably 0.3 mg/mL to 0.4 mg/mL (e.g., polysorbate 80 with a concentration of 0.2 mg/mL to 0.4 mg/mL, or 0.25 mg/mL to 0.4 mg/mL, preferably 0.3 mg/mL to 0.4 mg/mL). In some embodiments, the pharmaceutical preparation is a liquid injection (e.g., a liquid in vial, a liquid in ampoule or a liquid in prefilled syringe) or lyophilized powder.

In some embodiments, the pharmaceutical preparation has a pH of 5.8 to 6.5, 5.9 to 6.5, 6.0 to 6.5, 6.0 to 6.4, 6.0 to 6.3, or 6.0 to 6.2, for example, a pH of 6.0, 6.1, 6.2, 6.3, 6.4, or 6.5.

In some embodiments, the pharmaceutical preparation comprises: 40 mg/mL to 80 mg/mL the anti-VEGFA/-VEGFC bispecific binding protein, 0.80 mg/mL to 1.0 mg/mL histidine, 0.80 mg/mL to 1.0 mg/mL L-histidine hydrochloride, 80 mg/mL to 100 mg/mL sugar, and 0.2 mg/mL to 0.5 mg/mL polysorbate, the pharmaceutical preparation having a pH of 6.0 to 6.5.

In some embodiments, the pharmaceutical preparation comprises: 40 mg/mL to 80 mg/mL the anti-VEGFA/-VEGFC bispecific binding protein, 0.80 mg/mL to 1.0 mg/mL histidine, 0.80 mg/mL to 1.0 mg/mL L-histidine hydrochloride, 80 mg/mL to 100 mg/mL trehalose, and 0.2 mg/mL to 0.5 mg/mL polysorbate 80, the pharmaceutical preparation having a pH of 6.0 to 6.5.

In some embodiments, the pharmaceutical preparation comprises: 40 mg/mL to 60 mg/mL the anti-VEGFA/-VEGFC bispecific binding protein, 0.85 mg/mL to 0.95 mg/mL histidine, 0.85 mg/mL to 0.95 mg/mL L-histidine hydrochloride, 85 mg/mL to 100 mg/mL trehalose, and 0.2 mg/mL to 0.4 mg/mL polysorbate 80, the pharmaceutical preparation having a pH of 6.0 to 6.2.

In some embodiments, the pharmaceutical preparation comprises: 50 mg/mL the anti-VEGFA/VEGFC bispecific binding protein, 0.90 mg/mL histidine, 0.90 mg/mL L-histidine hydrochloride, 90.00 mg/mL trehalose, and 0.3 mg/mL polysorbate 80, the pharmaceutical preparation having a pH of 6.2.

In some embodiments, the pharmaceutical preparation has an osmotic pressure of 280 mOsmol/kg or more, preferably 300 mOsmol/kg or more, for example, 300 mOsmol/kg to 350 mOsmol/kg.

In some embodiments, the pharmaceutical preparation keeps stable at 30°C to 50°C for more than 2 weeks, preferably more than 4 weeks, in a form of liquid or lyophilized powder. The stability of the pharmaceutical preparation herein may be determined by measuring protein content using a UV method, by measuring purity using an SEC-HPLC method or a non-reducing CE-SDS method, by detecting visible foreign matters using a visible foreign matter inspection method, by detecting appearance using an observation method, and/or by measuring the pH of the preparation using a pH determination method, but not limited to these methods. In some embodiments, the pharmaceutical preparation, when stored at 30°C to 50°C for more than 2 weeks in a form of liquid, maintains a purity of more than 55%, more than 65%, more than 70%, more than 75%, or more than 80% as determined by an SEC-HPLC method.

In another aspect, the present application relates to use of the pharmaceutical preparation described above in preparation of a medicament for treating neovascularization-related diseases or a reagent for diagnosing neovascularization-related diseases. Or, the present application relates to a pharmaceutical preparation for use in treatment or diagnosis of neovascularization-related diseases. Or, the present application relates to a method for treating or diagnosing neovascularization-related diseases, comprising administrating the pharmaceutical preparation described above to a subject in need thereof. Or, the present application relates to a pharmaceutical product for use in treatment or diagnosis of neovascularization-related diseases, wherein the pharmaceutical product comprises the pharmaceutical preparation described above.

In some embodiments, the diseases comprise solid tumors or ophthalmic diseases. In some preferred embodiments, the diseases comprise solid tumors, preferably melanoma, wherein the pharmaceutical preparation may be used to inhibit neovascularization in tumors and/or tumor growth. In further preferred embodiments, the diseases comprise ophthalmic diseases, preferably age-related macular degeneration, diabetic retinopathy, retinal vascular occlusion and corneal neovascularization.

In another aspect, the present application relates to a kit, comprising the pharmaceutical preparation described above.

In some embodiments, the kit further comprises: one or more additional activators, which may be further administrated in combination with the pharmaceutical preparation of the present application. The mode of administrating in combination may comprise simultaneous administrating, parallel administrating, or successive administrating in any order.

In some embodiments, the activator may be any one of: a chemotherapeutic agent, a radiotherapeutic agents, an anti-neovascularization drug, an immunosuppressive drug, an anti-fibrotic drug, a neuroprotective agent, and drugs with tumor-inhibiting effects.

In some embodiments, the kit further comprises a device for administration to a subject. In some embodiments, the device for administration to a subject is a syringe (e.g., a prefilled syringe, a needleless syringe or an injection pen), an inhalation device, an implantation device, or an infusion device.

In some embodiments, the kit further may further comprise, for example, an instruction for use; other reagents, for example, a marker or a coupling reagent; and/or a pharmaceutical carrier.

In some embodiments, the bispecific binding protein of the present application or the pharmaceutical preparation, medicament or kit comprising the same may achieve any one or more biological activities selected from:

(i) blocking the activation of a VEGFR2 signaling pathway induced solely by hVEGFA, wherein preferably, an inhibitory activity IC50 value may be determined using a KDR reporter molecule assay, with the IC50 value of for example 0.1 nM to 10 nM, for example, about 0.2 nM to 1 nM (such as about 0.6 nM), or about 1 nM to 3 nM (such as about 1 nM);

(ii) blocking the activation of a VEGFR2 signaling pathway induced solely by hVEGFC, wherein preferably, the inhibitory activity IC50 value may be determined using a KDR reporter molecule assay, with the IC50 value of 0.1 nM to 1 nM, for example about 0.2 nM to 0.6 nM;

(iii) inhibiting lymphatic cell proliferation induced by hVEGFC, wherein preferably, the inhibitory activity IC50 value may be determined using a cell proliferation assay, with the IC50 value of 0.01 nM to 0.5 nM, for example about 0.1 nM to 0.5 nM;

(iv) inhibiting endothelial cell survival and proliferation induced by both hVEGFC and hVEGFA, with an inhibition level up to at least 80%, 85%, 90%, 95% or about 100%, wherein preferably, the inhibitory activity IC50 value may be determined using the cell proliferation assay, with the IC50 value of 0.1 nM to 0.5 nM, for example about 0.2 nM;

(v) inhibiting neovascular structure formation induced by both hVEGFC and hVEGFA, with an inhibition level up to at least 80%, 85%, 90%, 95% or about 100%, wherein preferably, an inhibitory activity may be determined using an endothelial cell tube formation assay, for example, the method described in Example 13;

(vi) inhibiting neovascularization in tumors (e.g., solid tumors, such as melanoma);

(vii) inhibiting growth of tumors (e.g., solid tumors, such as melanoma), for example, to achieve a tumor inhibition rate up to more than 50% with monotherapy in a tumor-bearing animal model; and

(viii) preventing and inhibiting the occurrence and/or progression of neovascularization-related ophthalmic diseases, for example, to reduce the neovascularization level, decrease vascular leakage, and inhibit fundus retinal edema and/or thickening caused by neovascularization.

The pharmaceutical preparation, medicament and kit according to the present application can be applied in vivo and/or in vitro to:

- bind VEGFA antigens and/or VEGFC antigens;
- block the binding of VEGFA and/or VEGFC to their associated receptors, for example, VEGFR2 and/or VEGFR3;
- inhibit the activation of VEGFR2 and/or VEGFR3 cell signaling as induced by VEGFA and/or VEGFC;
- inhibit vascular endothelial cell survival, proliferation, and/or migration as induced by VEGFA and/or VEGFC;
- inhibit lymphocyte survival and/or proliferation as induced by VEGFC;
- inhibit lymphangiogenesis and lymphatic endothelial cell growth and migration as induced by VEGFC;
- inhibit neovascularization and/or vascular leakage as induced by VEGFA and/or VEGFC; and
- inhibit tumor neovascularization and/or growth and/or metastasis.

## BRIEF DESCRIPTION OF THE DRAWINGS

Fig. 1 shows an assay of blocking VEGFR3 by affinity-matured anti-VEGFC VHH;

Fig. 2 shows an assay of blocking the VEGFC-induced activation of HEK 293 KDR reporter molecules by an affinity-matured molecule, anti-VEGFC VHH antibody;

Fig. 3 shows an assay of blocking VEGF-C-induced BaF3-FLT4 proliferation by an affinity-matured anti-VEGFC VHH antibody;

Fig. 4 shows an assay of blocking the VEGFA-induced activation of HEK 293 KDR reporter molecules by bispecific antibody molecules;

Fig. 5 shows an assay of blocking the VEGFC-induced activation of HEK 293 KDR reporter molecules by bispecific antibody molecules;

Fig. 6 shows an assay of inhibiting VEGFC-induced BaF3-FLT4 proliferation by bispecific antibody molecules;

Fig. 7 shows an assay of inhibiting VEGFA+C-induced HUVEC proliferation by bispecific antibody molecules;

Fig. 8A to Fig. 8B show an assay of inhibiting VEGFA+C-induced HUVEC lumen formation by bispecific antibody molecules, with Fig. 8A showing a lumen formation image, and Fig. 8B showing lumen formation statistics;

Fig. 9A to Fig. 9C show an assay of inhibiting neovascularization in an A375 subcutaneous tumor model by bispecific antibody molecules, with Fig. 9A showing the mass statistics of A375 tumor, Fig. 9B showing the volume statistics of A375 tumor, and Fig. 9C showing the CD31 staining image of A375 tumor;

Fig. 10 shows an assay of inhibiting laser-induced CNV by bispecific antibody molecules;

Fig. 11 shows a fundus fluorescein angiography (FFA) graph of laser-induced choroidal neovascularization inhibited by an anti-VEGFA/VEGFC bispecific antibody;

Fig. 12 shows an OCT graph of laser-induced retinal thickening inhibited by the anti-VEGFA/VEGFC bispecific antibody;

Fig. 13A to Fig. 13B show pathological changes in a laser-induced choroidal neovascularization model inhibited by the anti-VEGFA/VEGFC bispecific antibody;

FIG. 14 shows a schematic structural diagram of a bispecific antibody constructed using a VEGF-A binding domain-Fc fusion protein in combination with an anti-VEGF-C VHH antibody as a component;

FIG. 15 shows original SEC-HPLC profiles of samples kept at different pHs for 2 weeks;

FIG. 16 shows original non-reduced CE-SDS profiles of samples kept at different pHs for 2 weeks;

Fig. 17 shows a trend chart of monomer and polymer changes in purity (SEC-HPLC method) of samples with different formulations; and

FIG. 18 shows a trend chart of monomer, polymer and fragment changes in purity (non-reduced CE-SDS method) of samples with different formulations.

## DETAILED DESCRIPTION OF THE PREFERRED EMBODIMETNS

The present application will be described in detail below by means of embodiments, which, however, do not imply the existence of any unfavorable limitations to the present application. The present application has been described in detail herein, with specific embodiments thereof disclosed as well. Without departing from the spirit and scope of the present application, various changes and improvements made to the specific embodiments of the present application shall also fall within the scope of protection of the present application for those skilled in the art.

Unless otherwise defined, the technical and scientific terms as used herein have the same meaning as those commonly understood by those of ordinary skill in the art to which the present disclosure belongs. See, for example, Singleton et al, Dictionary of Microbiology and Molecular Biology 2nd ed., J. Wiley & Sons (New York, NY 1994); Sambrook et al, Molecular Cloning, A Laboratory Manual, Cold Springs Harbor Press (Cold Springs Harbor, NY 1989); Current Protocols in Molecular Biology or Current Protocols in Immunology, John Wiley & Sons, New York, N.Y. (2009); Perbal, A Practical Guide to Molecular Cloning (1984).

Unless otherwise clearly indicated in the context, the term "or" is intended to include "and" herein, and vice versa. Unless otherwise specified, a singular term covers plural referents herein, and vice versa.

Unless otherwise specified, the terms "comprise, comprises and comprising" or their equivalents (e.g., contain, containing, include, and including) are open-ended herein and should be understood as "including but not limited to", meaning that other unspecified elements, components and steps may be covered in addition to the elements, components and steps as listed.

The term "about", when used in conjunction with a numerical value, is intended to cover numerical values within a range having a lower limit of 5% less than the specified numerical value and an upper limit of 5% greater than the specified numerical value.

As used herein, the terms "fusion protein" and "chimeric polypeptide" are used interchangeably to refer to a larger polypeptide formed by the fusion of at least two heterologous polypeptide sequences, optionally via a linker. The fusion protein may be produced by recombinant expression.

As used herein, the term ligand "trap" molecule is a fusion protein that comprises a ligand-interacting extracellular domain of a receptor and a human IgG Fc region. At present, a variety of trap molecules have been developed to "trap" VEGF ligands, including VEGFA and VEGFC. These trap molecules may be used to bind to corresponding ligands in an extracellular environment and reduce their concentrations. In an embodiment, the anti-VEGFA component of the present application comprises a trap molecule that "traps" VEGFA and is in the form of an Fc fusion protein. The trap molecule can compete with natural VEGFA cellular receptors for binding to VEGFA, thereby inhibiting VEGFA-induced signaling. In an embodiment, the trap molecule in the form of Fc fusion protein comprises an amino acid sequence as set forth in SEQ ID NO: 5. This VEGFA-trap molecule is formed by the fusion of extracellular ligand-binding domains from VEGF receptors 1 and 2 to the Fc moiety of human IgG1, and it has amino acids derived exclusively from human, thereby minimizing the immunogenicity of the molecule in human.

The term "VHH domain" as used herein is intended to refer to a heavy chain variable domain derived from a heavy chain antibody lacking a light chain (sometimes referred to as an HcAb antibody herein), and it is also referred to as a single variable domain fragment or nanobody. Hence, the VHH domain differs from the conventional VH domain of a four-chain immunoglobulin in that it does not need to be paired with a light chain variable domain to form an antigen-binding site. Such VHH domain molecules may be derived from antibodies produced in Camelidae species (e.g., camels, alpacas, dromedaries, llamas, and guanacos). Species other than Camelidae species may also produce heavy chain antibodies that naturally lack a light chain, and these VHHs shall also fall within the scope of the present application. In some cases, the therapeutic application of the VHH domain is desired to reduce its immunogenicity. Therefore, preferably, in an embodiment, the VHH domain used in the present application is a humanized VHH domain or a further sequence-optimized form thereof (e.g., in an affinity-matured form with an increased binding affinity).

"Single-domain antibody" or 'sdAb' as used herein is intended to refer to an antibody polypeptide that recognizes and binds to a target antigen via a single variable domain (e.g., VHH or a single VH or a single VL) of an antibody. The single variable domain of a single-domain antibody is capable of recognizing and binding to a target antigen, without the need of being paired with another antibody's variable domain. A single-domain antibody comprising a heavy chain variable domain (VHH) of a heavy chain antibody is also referred to as a VHH single-domain antibody herein. The VHH single-domain antibody used in the present application is preferably derived from a Camelidae animal (for example, alpaca), or a humanized or sequence-optimized form thereof. In some embodiments, the VHH single-domain antibody of the present application is a monovalent monospecific polypeptide molecule consisting of or substantially consisting of a single VHH domain.

The "complementarity determining region" or "CDR region" or "CDR" or "highly variable region" of an antibody is a region in an antibody variable domain (VH or VHH) that is highly variable in sequence and forms a structurally defined loop ("hypervariable loop") and/or comprises antigenic contact residues ("antigenic contact sites"). The CDR is mainly responsible for binding to an antigenic epitope. The CDRs of the heavy and light chains are numbered in sequence from the N-terminus and are commonly referred to as CDR1, CDR2, and CDR3. CDRs located within the variable domains of an antibody's heavy chain are also referred to as HCDR1, HCDR2, and HCDR3, and CDRs located within the variable domains of an antibody's light chain are referred to as LCDR1, LCDR2, and LCDR3. In a given amino acid sequence of a light chain variable region or heavy chain variable region, its CDR sequence may be determined using a variety of schemes known in the art, for example, Chothia based on the three-dimensional structure of the antibody and the topology of the CDR loop, Kabat based on the variability of the antibody sequence (Kabat et al, Sequences of Proteins of Immunological Interest, 4th edition, U.S. Department of Health and Human Services, National Institutes of Health (1987)), AbM (University of Bath), Contact (University College London), International Immunogenetics Database (IMGT) (International Immunogenetics Information System, World Wide Web imgt.cines.fr/), and the North CDR definition of affinity propagation clustering based on the use of a large number of crystal structures (North et al., "A New Clustering of Antibody CDR Loop Conformations", Journal of Molecular Biology, 406, 228-256 (2011)).

The following provides the regional ranges of CDRs defined using the kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |

(continued)

| CDR | Kabat scheme | AbM scheme | Chothia scheme | Contact scheme | IMGT scheme |
|---|---|---|---|---|---|
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

Unless otherwise specified, in the present application, the term "CDR" or "CDR sequence" covers a CDR sequence determined in any of the above ways.

The CDR can also be determined based on the same Kabat numbered positions as the reference CDR sequence. Unless otherwise specified, in the present application, residue positions as mentioned in an antibody variable region (including residues in heavy chain variable regions and in light chain variable regions) refer to positions numbered based on the Kabat numbering system (Kabat et al, Sequences of Proteins of Immunological Interest, 5th Ed. Public Health Service, National Institutes of Health, Bethesda, Md. (1991)).

The "percent identity (%)" of an amino acid sequence refers to the percentage at which amino acids residues in an candidate sequence are identical to amino acid residues in a specific amino acid sequence shown in the present Specification, when the candidate sequence is aligned to the specific amino acid sequence shown in the present Specification and null positions are introduced, if necessary, to achieve the maximum percent sequence identity, without the consideration of any conserved substitution as part of the sequence identity. In some embodiments, the present application takes variants of the antibody molecules of the present application into consideration, and these variants have a considerable degree of identity (e.g., an identity of at least 80%, 85%, 90%, 95%, 97%, 98%, or 99%, or more) to the antibody molecules and their sequences specifically disclosed herein. The variants may comprise conserved modifications.

In the case of polypeptide sequences, the "conserved modifications" comprise substitutions, deletions or additions to the polypeptide sequence, which, however, do not substantially alter the desired functional activity of the polypeptide sequence. For example, the conserved substitution often leads to the substitution of an amino acid for a chemically similar amino acid. The table of conserved substitutions to provide functionally similar amino acids is well-known in the art. The following lists 8 groups of amino acids allowing for mutual conserved substitution: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conserved sequence modification" refers to an amino acid modification that does not significantly affect or alter the antigen binding characteristics of interest of the antibody molecule or binding protein molecule of the present application comprising an amino acid sequence. For example, the variants subjected to conserved modification maintain at least 80%, 85%, 90%, 95%, 98%, 99% or higher (e.g., 100% to 110% or higher) binding affinity for the antigen of interest with respect to the parent antibody or binding protein.

The terms "individual" or "subject" are used interchangeably to refer to mammals. The mammals comprise, but are not limited to, domesticated animals (e.g., dairy cattle, sheep, cats, dogs, and horses), primates (e.g., humans and non-human primates such as monkeys), rabbits, and rodents (e.g., mice and rats). Specifically, the individual is a human.

The term "treatment" refers to a clinical intervention intended to alter the natural course of a disease in an individual under treatment. Desired therapeutic effects comprise, but are not limited to, preventing the onset or recurrence of a disease, alleviating symptoms, reducing any direct or indirect pathological consequences of a disease, preventing metastasis, slowing the rate of disease progression, ameliorating or alleviating the disease state, and alleviating or improving prognosis. In some embodiments, the antibody molecule of the present application is used to slow the development of disease or the progression of the disease.

The term "anti-tumor effect" or "tumor suppression effect" refers to biological effects that can be exhibited by various means, including but not limited to, for example, reduced tumor volume, reduced number of tumor cells, reduced tumor cell proliferation, or reduced tumor cell survival. The terms "tumor" and "cancer" are used interchangeably herein to encompass various solid tumors.

The term "neovascularization-related diseases" herein refers to diseases, disorders, and/or conditions with the occurrence, development, and/or progression involving the occurrence of neovascularization (including angiogenesis, lymphangiogenesis, and/or both). Such diseases, disorders, or conditions would benefit from the blocking of biological activities of VEGF-C or VEGF-A or both.

FR1 to FR4 herein refer to framework regions 1 to 4; and CDR1 to CDR3 refer to complementarity determining regions 1 to 3.

The bispecific binding protein or its variants as described herein has/have dual antagonistic activities, and can inhibit the binding of VEGFA to its VEGF receptor and inhibit the binding of VEGFC to its VEGF receptor.

Pharmaceutical stability is one of the important indicators for ensuring the effectiveness and safety of pharmaceuticals. To keep the safety and efficacy of the pharmaceuticals during their shelf life, it is a crucial condition to acquire a preparation formulation endowing the pharmaceuticals with good stability. Next, the formulation is evaluated and determined by selecting buffer pH and excipients through high-temperature stability assays, inspecting appearance and visible foreign matters, and measuring protein content, purity, charge variants, polysorbate 80 content, and biological activity, to obtain a formulation with good stability.

**Examples**

Experimental methods without clear indication of specific conditions are all carried out following conventional conditions. Unless otherwise specified, the reagents, materials, or instruments used in the following examples without clear indication of manufacturers are all commercially available conventional products.

**Example 1: Protein Expression and Purification for Anti-VEGFC Humanized VHH Antibody**

Plasmids comprising nucleic acids encoding an anti-VEGFC antibody am63G12.1-5G8-18B9 (VHH sequence SEQ ID NO: 4, CDR1: SEQ ID NO: 1; CDR2: SEQ ID NO: 2; CDR3: SEQ ID NO: 3, with CDR1 defined according to the combination of Kabat and Chothia, and CDR2 and CDR3 defined according to Kabat) were prepared for transfection of 293 cells and expression of VHH single-domain antibodies or VHH-Fc antibodies. Expi-293 cells (Invitrogen) were passaged based on the desired transfection volume, and the cell density was adjusted to $1.5 \times 10^6$ cells/mL the day before transfection. On the day of transfection, the cell density was approximately $3 \times 10^6$ cells/mL. An F17 medium (Gibco, A13835-01) being 1/10 of the final volume was taken as a transfection buffer, to which appropriate plasmids were added and mixed well. Appropriate polyethyleneimine (PEI) (Polysciences, 23966) was added to the plasmids (the ratio of plasmids to PEI was 1:3 in the 293F cells), mixed well and incubated at room temperature for 10 min to obtain a DNA/PEI mixture. The cells were resuspended with the DNA/PEI mixture and then incubated for 24 h at 36.5°C in the presence of 8% $CO_2$, followed by the supplementation of FEED (Sigma) that was 2% of the transfection volume, and the resulting mixture was incubated at 36.5°C and at 120 rpm in the presence of 8% $CO_2$. After continuous incubation until Day 6 or when the cell viability was $\leq 60\%$, the cell supernatant was collected for purification. Before purification, the collected medium was centrifuged at 4500 rpm for 30 min, and the cells were discarded. The supernatant was then filtered using a 0.22 $\mu$m filter. The Protein A column (Hitrap Mabselect Sure 5*5ml, GE, 11-0034-95) was equilibrated using 10 mL of a binding buffer (20 mM sodium phosphate, 150 mM NaCl, pH 7.0). The filtered supernatant was added to the purification column, which was then re-equilibrated using 15 mL of the binding buffer. 5 mL of an elution buffer (citric acid + sodium citrate 0.1 M, pH 3.5) was added, the eluate was collected, and 80 $\mu$L of Tris-HCl was added per 1 mL of eluate. The collected antibodies were ultrafiltered, concentrated and then exchanged into PBS (Gibco, 70011-044), and the concentration was measured.

**Example 2: Determination of Binding Kinetics of Antibodies of the Present Application to Antigens by Biofilm Thin Layer Interferometry**

The equilibrium dissociation constant ($K_D$) for the antibodies of the present application bound to human VEGFC was determined using the biofilm thin layer interferometry (ForteBio). The ForteBio affinity determination was carried out according to an existing method (Estep, P et al. High throughput solution based measurement of antibody-antigen affinity and epitope binning. MAbs, 2013.5(2): pp. 270-8).

Half an hour before the start of the assay, depending on the number of samples (anti-VEGFC antibodies am63G12.1-5G8-18B9), an appropriate number of AMQ (Pall, 1506091) (for sample detection) or AHQ (Pall, 1502051) (for positive control detection) sensors were immersed in an SD buffer ($1 \times$PBS, 0.1% of BSA, 0.05% of Tween-20).

100 $\mu$L of the SD buffer, antibodies, and antigens (human VEGFC (SinoBiological)) were added to a 96-well black polystyrene half-dose microwell plate (Greiner, 675076). The plate was set up based on sample position and sensor positions were selected. The instrument was set up with the following parameters: operating steps: Baseline, Loading ~1 nm, Baseline, Association, and Dissociation; the run time of each step depended on the speed of sample binding and dissociation, with a rotational speed of 400 rpm and a temperature of 30°C. $K_D$ values were analyzed using ForteBio analysis software.

The affinities of the antibodies in the assay described above are shown in Table 1:

Table 1. Affinity constants (equilibrium dissociation constants) for monovalent antigen-antibody binding in ForteBio assay

| Antibody | Antigen | $K_D$ (M) | $K_{on}$(1/Ms) | $k_{dis}$(1/s) |
|---|---|---|---|---|
| Am63G12.1-5G8-18B9 | VEGFC | 5.70E-09 | 6.31E+04 | 3.60E-04 |

In the above assay, the binding affinity of the affinity-matured antibody Am63G12.1-5G8-18B9 was further increased, with a monovalent $K_D$ value of 5.70E-09 M.

## Example 3: ELISA Blocking Assay of Anti-VEGFC VHH Antibody

The effect of the antibody of the present application on blocking the binding of hVEGFC to the receptor KDR was determined.

SA (streptavidin) was diluted to 1 μg/mL and seeded at 100 μL/well to an ELISA plate, which was left at 4°C overnight. The plate was washed 3 times with PBST and blocked for 1.5h by the addition of 3% BSA. The plate was washed 3 times with PBST, and 50 ng/mL VEGFC-biotin (biotin-labeled human VEGFC) was added and incubated for 1.5h. 50 μL of the antibody was incubated with VEGFR2-Fc (final concentration: 0.2 μg/mL, SinoBiological) or VEGFR3-Fc (final concentration: 0.2 μg/mL, SinoBiological) for 20 min in advance and then added to the plate. The plates were washed 3 times with PBST, and an anti-human FcHRP antibody (diluted at 1:10,000, BETHYL) was added and incubated for 30 min. The plates were washed 6 times with PBST, TMB (SOLARBIO) was used to develop the color for 5 min, and after termination, reading was carried out at OD 450nm.

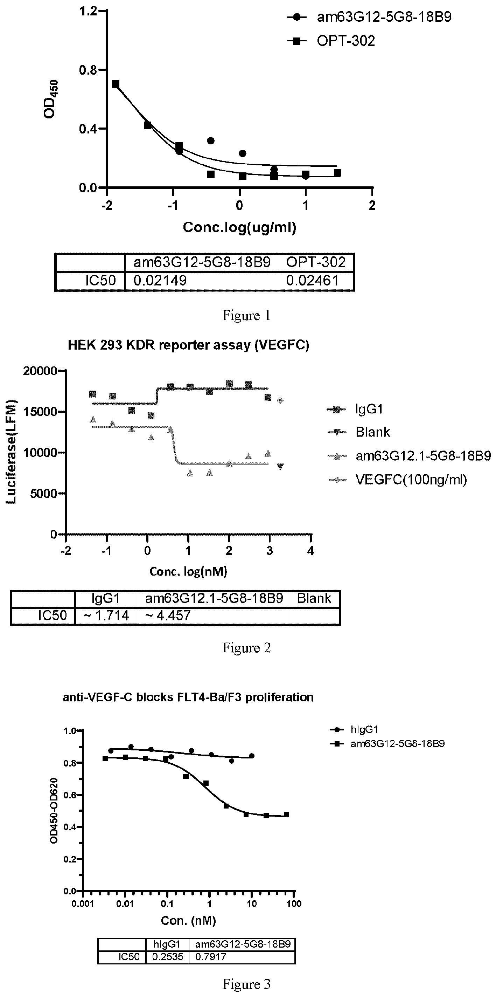

The blocking results of the anti-VEGFC VHH antibody obtained in the present application were shown in Fig. 1. The candidate molecule, Am63G12.1-5G8-18B9 antibody, could block the binding of VEGFC to VEGFR3, and showed a blocking IC50 value substantially comparable to the positive control molecule, OPT-302 (VEGF-C-trap, SEQ ID NO: 9).

## Example 4: Assay of Blocking VEGFC-induced Activation of HEK293 KDR Reporter Molecules by Anti-VEGFC VHH Antibody

VEGFC can bind to the related receptor VEGFR2 (KDR) to activate the VEGFR2 signaling pathway and induce effects such as vascular endothelial cell survival, proliferation and migration. In this study, the effect of the gradient-diluted antibody on blocking the VEGFC-induced activation of the signaling pathway of the related receptor was tested with the KDR reporter molecule assay system using NFAT-RE-luc2P/KDR HEK293 cells (Promega Cat CS181401).

A reference was made to the supplier's instructions for experimental methods.

NFAT-RE-luc2P/KDR HEK293 cells that were transferred to an experimental medium (DMEM medium containing 10% FBS) 3 days in advance were removed; the used medium was aspirated; the cells were washed once with PBS, after which the cells were digested with 1 mL of Accutase solution (Sigma) until the cells were rounded and detached from the wall, the reaction was terminated using 5 mL of diluted medium (DMEM medium containing 10% FBS); the cells were sucked into a centrifuge tube, which was centrifuged at 1000 rpm for 5 min; the medium was discarded; 10 mL of diluted medium was added to resuspend the cells; and the cells were mixed well and counted, and the cell viability should be above 90%. The cell density was adjusted to $0.8 \times 10^6$ cells/mL with the diluted medium, and added into 96-well white cell culture plate at 50 μL/well according to the experimental layout.

A mixture of hVEGFC (R&D) with a concentration of 200 ng/mL and a gradient-diluted antibody to be tested (comprising the VHH antibody of the present application, a positive control molecule (OPT-302), and a negative control antibody (IgG isotype control antibody)) was prepared, allowed to stand for 30 min, and then added at 50 μL/well to the 96-well white cell culture plate containing the cells; and the plate was incubated in a 5% carbon dioxide incubator at 37°C. At the same time, groups were set up as follows: a blank control group in which the antibody and VEGFC were not added; and a VEGF-C experimental group in which only VEGFC was added, without any antibody.

The 96-well white cell culture plate that had been incubated for 6 h was removed from the carbon dioxide incubator and equilibrated for 10 min to 15 min to room temperature. Bio-Glo luciferase assay system (Promega) that had been equilibrated to room temperature in advance was added to the 96-well white cell culture plate at 100 μL/well according to the experimental layout, and incubated at room temperature in the dark for 5 min. The fluorescence reading was performed using a multifunctional microplate reader, and by selecting chemiluminescence mode for plate reading mode and endpoint method for plate reading type and setting the wavelength to full wavelength, the fluorescence was collected column by column, for 1000 ms for each column. The results were shown in Fig. 2, indicating that the Am63G12.1-5G8-18B9 antibody could completely inhibit the VEGFC-induced activation of the KDR signaling pathway, with the IC50 of about 4.457 nM, which was even lower than that of its parental antibody (data not shown).

**Example 5: Assay of Inhibiting VEGFC-induced BaF3-FLT4 Cell Proliferation by Anti-VEGFC VHH Antibody**

In this study, BaF3 cells overexpressing FLT4 (VEGFR3) (Institute of Basic Medical Sciences, Chinese Academy of Medical Sciences), BaF3-FLT4, were used to co-incubate the antibodies and the recombinant human VEGFC proteins. The number of viable cells was detected by a CCK-8 kit (Dojindo), which reflected the inhibitory effect of different antibodies on VEGFC-induced BaF3-FLT4 proliferation.

The BaF3 cells BaF3-FLT4 overexpressing FLT4 were obtained by infecting BaF3 cells with lentiviruses carrying FLT4 genes.

A cell proliferation inhibition assay was carried out according to the instructions of CCK-8 kit. An experimental medium was prepared using a 1640 medium containing 10% FBS. The highest final concentration of the test antibodies (comprising the VHH antibody of the present application, a positive control molecule (OPT-302) and a negative control antibody (IgG isotype control antibody)) was 20 $\mu$g/mL, which was serially diluted 1:3 at equal ratio. At the same time, groups were set up as follows: a blank control group in which antibody and VEGFC were not added; and a VEGF-C experimental group in which only VEGFC was added, without any antibody. In the test system, the final concentration of hVEGFC (R&D) was 20 ng/mL and the final concentration of BaF3-FLT4 cells was $2*10^5$ cells/mL. 100 $\mu$L of the system was added to each well of a 96-well plate, which was then incubated for 72 h in a $CO_2$ incubator at 37°C.

Afterwards, 15 $\mu$L of CCK-8 was added to each well and incubated for 4 h in the $CO_2$ incubator at 37°C. The absorbance values were determined using dual wavelengths, with the detection wavelength of 450 nm, and the reference wavelength of 620 nm.

The OD450-OD620 values were determined.

The experimental results were shown in Fig. 3. The Am63G12.1-5G8-18B9 antibody of the present application can effectively inhibit the hVEGFC-induced BaF3-FLT4 survival and proliferation in vitro, with the IC50 of 0.7917 nM, which is even lower than that of its parental antibody (data not shown).

**Example 6: Construction of Anti-VEGFA/VEGFC Bispecific Antibody**

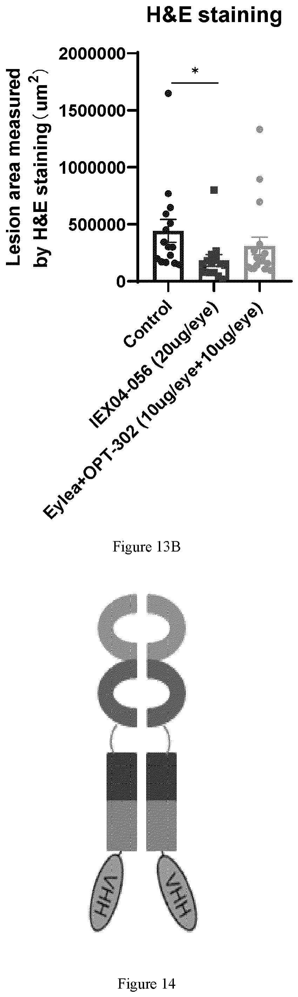

The anti-VEGFA/VEGFC bispecific antibody was constructed with am63G12.1-5G8-18B9 and eukaryotic protein expression was carried out in 293 cells according to Example 1. In brief:
the bispecific antibody comprising a first polypeptide chain and a second polypeptide chain as shown in Fig. 14 was constructed by linking the VHH single-domain antibody to the C-terminus of the Fc region of the VEGF-Trap (Aflibercept, Eylea) via a linker. The VEGF-Trap was a recombinant fusion protein (SEQ ID NO: 5) formed by the fusion of human IgG1 Fc regions (SEQ ID NO: 7) at the C-termini of the VEGF-A binding domains (SEQ ID NO: 6) from human VEGF receptors 1 and 2; and it was in a dimer form to provide high-affinity VEGF-A binding and block the VEGF-A-induced activation of the VEGFR signaling pathway.

Table 2 below lists the constructed bispecific antibody and its composition.

Table 2. Bispecific antibody and its composition

| Bispecific antibody | Anti-VEGFA component | Linker | Anti-VEGF-C component | Sequence of the first/second polypeptide chain forming the antibody |
|---|---|---|---|---|
| IEX04-056 | Aflibercept | G(G$_4$S)$_3$ | am63G12.1-5G8-18B9 | SEQ ID NOs: 8/8 |

**Example 7: Assay of Blocking VEGFA- or VEGFC-induced Activation of HEK293 KDR Reporter Molecule by Anti-VEGFA/VEGFC Bispecific Antibody**

VEGFA or VEGFC can bind to the related receptor VEGFR2 (KDR) to activate the VEGFR2 signaling pathway and induce effects such as vascular endothelial cell survival\proliferation and migration. In this study, the blocking effect of the gradient-diluted antibodies on the VEGFA- and VEGFC-induced activation of the relevant receptor signaling pathway was tested by utilizing the KDR reporter molecular assay system and using NFAT-RE-luc2P/KDR HEK293 cells (Promega Cat CS181401). The experimental method were performed substantially as described in Example 4. In brief, a 96-well white cell culture plate containing NFAT-RE-luc2P/KDR HEK293 cells ($0.8\times10^6$ cells/mL, 50 $\mu$L/well) was prepared. A mixture of hVEGFA (R&D) with a concentration of 100 ng/mL or hVEGFC (R&D) with a concentration of 200 ng/mL and gradient-diluted antibodies to be tested was prepared and allowed to stand for 30 min. The mixture was added at 50 $\mu$L/well to the 96-well white cell culture plate containing NFAT-RE-luc2P/KDR HEK293 cells, and the plate was then incubated for 6 h in a 5% CO2 incubator at 37°C. At the same time, groups were set up as follows: a blank control group in which the antibody and VEGFC were not added; an experimental group in which only VEGFC was added, without any antibody was added; and an experimental group in which VEGFC and an IgG isotype control antibody were added. As a comparison, the activities of the anti-VEGFA molecule IBI304 (SEQ ID NO. 10) and the bispecific antibody Faricimab (anti-Ang-2/anti-VEGF-A) in blocking VEGF-A signaling pathway were also tested; and the activity of the anti-VEGF-C molecule OPT-302 in blocking VEGF-C signaling pathway was also tested.

The 96-well white cell culture plate that had been incubated for 6 h were taken out from the CO2 incubator and equilibrated for 10 min to 15 min to room temperature. Bio-Glo luciferase assay system (Promega) that had been equilibrated to room temperature in advance was added to the 96-well white cell culture plate at 100 $\mu$L/well according to the experimental layout, and incubated at room temperature in the dark for 5 min.

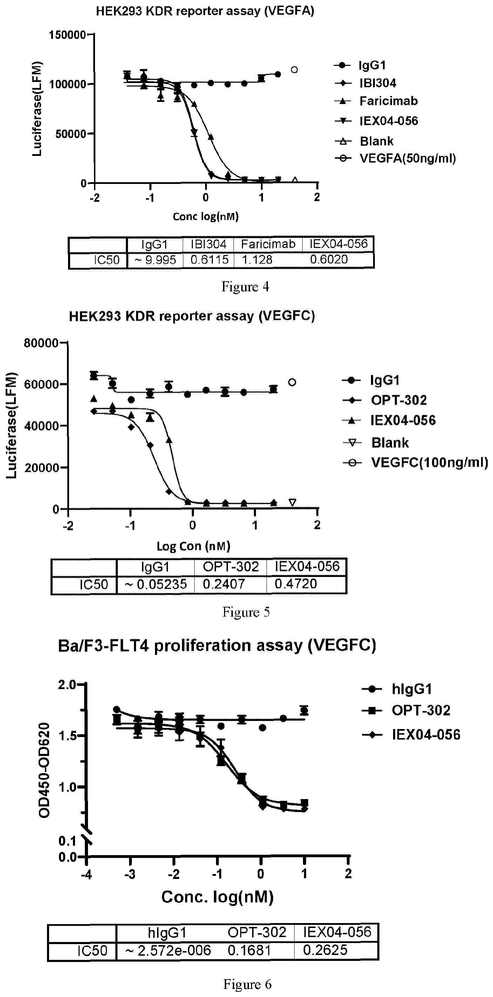

The fluorescence reading was performed using a multifunctional microplate reader, and by selecting chemiluminescence mode for plate reading mode and endpoint method for plate reading type and setting the wavelength to full wavelength, the fluorescence was collected column by column, for 1000 ms for each column. The test results are as shown in Fig. 4 and Fig. 5. Both the tested anti-VEGFA and anti-VEGFC bispecific antibodies can block the KDR signaling pathway activation induced by VEGFA (Fig. 4) or VEGFC (Fig. 5).

## Example 8: Assay of Inhibiting VEGFC-induced BaF3-FLT4 Proliferation by Anti-VEGFA/VEGFC Bispecific Antibody

The above-mentioned bispecific antibody of the present application was applied to the BaF3-FLT4 proliferation assay system to test the effect of the antibody on VEGFC-induced BaF3-FLT4 proliferation. The experimental method were performed substantially as described in Example 5.

An experimental medium was prepared using a 1640 medium containing 10% FBS. The highest final concentration of the test antibody was 10 nM, which was serially diluted at 1:3 in equal proportions. In the test system, the final concentration of hVEGFC (R&D) was 20 ng/mL and the final concentration of BaF3-FLT4 cells was $2*10^5$ cells/mL.

During determination, groups were set up as follows: a blank control group in which the antibody and VEGFC were not added; an experimental group in which only VEGFC was added, without any antibody was added; and an experimental group in which VEGFC and an IgG isotype control antibody were added. Also as a comparison, the inhibitory activity of the anti-VEGF-C molecule OPT-302 on VEGFC-induced BaF3-FLT4 cell proliferation was also tested.

The assay results are shown in Fig. 6, indicating that both the tested anti-VEGFA/VEGFC bispecific antibodies can inhibit the VEGFC-induced BaF3-FLT4 cell proliferation.

## Example 9: Assay of Inhibiting VEGFA+VEGFC-induced HUVEC Proliferation by Anti-VEGFA/VEGFC Antibody

VEGFA and VEGFC can act on VEGFR and other related receptors in vascular endothelial cells to promote vascular endothelial cell survival, proliferation and migration and thus induce neovascularization. In this assay, VEGFA and VEGFC were used to jointly induce human umbilical vein endothelial cell (HUVEC) survival and proliferation, to test the inhibitory effect of the antibody on the primary cell survival and proliferation induced by VEGFA and VEGFC. In this example, the survival and proliferation of HUVEC were determined by CCK-8 as follows. The cells were treated one day in advance, seeded to a 96-well culture plate at 2,000 cells/well and incubated in a 5% CO2 incubator at 37°C for 24 hours. After the cells were adhered to the wall, an experimental medium (DMEM medium) with or without VEGFA with a final concentration of 5 ng/mL and VEGFC with a final concentration of 50 ng/mL and/or gradient-diluted antibodies was prepared, the endothelial cell medium in the 96-well plate was replaced, and the plate was incubated in a 5% CO2 incubator at 37°C for 24 hours. Experimental groups were as follows:

Blank group: DMEM medium
VEGFA group: DMEM medium + 5 ng/mL VEGFA
VEGFC group: DMEM medium + 50 ng/mL VEGFC
VEGFA + VEGFC group: DMEM medium + 5 ng/mL VEGFA + 50 ng/mL VEGFC
IgG group: DMEM medium + 5 ng/mL VEGFA + 50 ng/mL VEGFC + isotype control IgG
IBI304 + OPT-302 group: DMEM medium + 5 ng/mL VEGFA + 50 ng/mL VEGFC + IBI304+ OPT-302 (IBI304 and OPT-302 were combined at a molar concentration ratio of 1:1);
Bispecific antibody group: DMEM medium + 5 ng/mL VEGFA + 50 ng/mL VEGFC + bispecific antibody to be tested.

After the cells were incubated with the experimental medium, a CCK-8 test solution (Dojindo) was added at 10 $\mu$L/well, and the cells were incubated in a 5% CO2 incubator at 37°C for 12 h to 24 h. The absorbance $OD_{450}$-$OD_{620}$ reading was carried out using a multifunctional microplate reader.

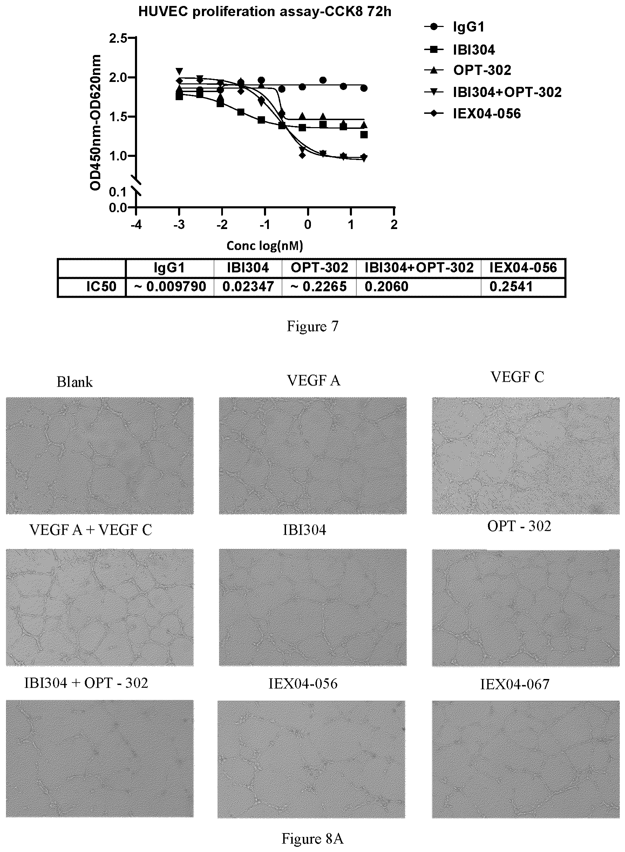

In the experiment described in the above assay, the results are shown in Fig. 7. The tested anti-VEGFA and anti-VEGFC bispecific antibodies were able to completely inhibit VEGFA + VEGFC-induced HUVEC cell proliferation and survival.

**Example 10: Assay of Inhibiting VEGFA+VEGFC-induced HUVEC Lumen Formation by Anti-VEGFA/VEGFC Antibody**

VEGFA and VEGFC can act on vascular endothelial cells to promote the formation of lumen-like structures from vascular endothelial cells and in turn induce the formation of neovascular structures. In this assay, VEGFA and VEGFC were used to induce the lumen formation from human umbilical vein endothelial cells (HUVECs), to test the inhibitory effect of antibodies on VEGFA- and VEGFC-induced primary cell lumen formation.

In this example, the inhibition of HUVEC lumen formation by the anti-VEGFA/VEGFC bispecific antibody was tested by a HUVEC lumen assay as follows. Matrigel (BD) was melted on ice one day in advance, and was added to a 96-well plate at 100 μL-well, and the plate was solidified in a $CO_2$ incubator at 37°C for half an hour. The cells were treated with an accutase solution, and then seeded to a 96-well culture plate at 20,000 cells/well, and the plate was incubated in a 5% CO2 incubator at 37°C for 24 h. An experimental medium (EGM2) with or without VEGFA with a final concentration of 5 ng/mL and VEGFC with a final concentration of 50 ng/mL and/or gradient-diluted antibodies was prepared. The HUVEC cells were resuspended using different experimental media, and then seeded to a 96-well culture plate at 20,000 cells/well, and the plate was incubated in a 5% CO2 incubator at 37°C for 24 h. Experimental groups were as follows:

> Blank group: EGM-2 medium
> VEGFA group: EGM-2 medium + 5 ng/mL VEGFA
> VEGFC group: EGM-2 medium + 50 ng/mL VEGFC
> VEGFA + VEGFC group: EGM-2 medium + 5 ng/mL VEGFA + 50 ng/mL VEGFC
> IgG group: EGM-2 medium + 5 ng/mL VEGFA + 50 ng/mL VEGFC + 20 nM isotype control IgG
> IBI304 + OPT-302 group: EGM-2 medium + 5 ng/mL VEGFA + 50 ng/mL VEGFC + 20 nM IBI304 + 20nM OPT-302
> IEX04-056 group: EGM-2 medium + 5 ng/mL VEGFA + 50 ng/mL VEGFC + 20 nM IEX04-056.

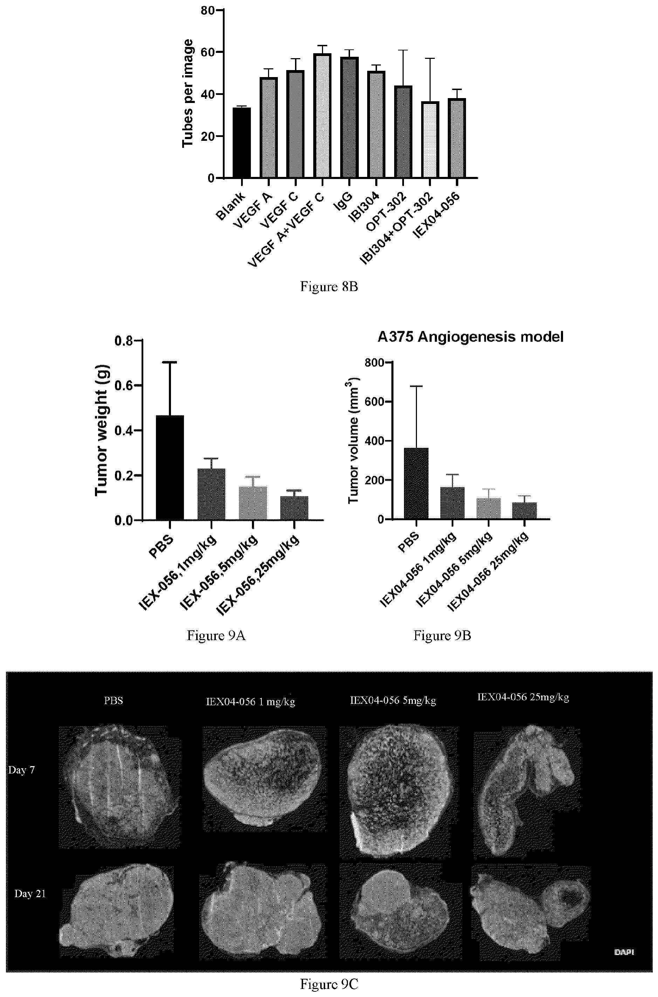

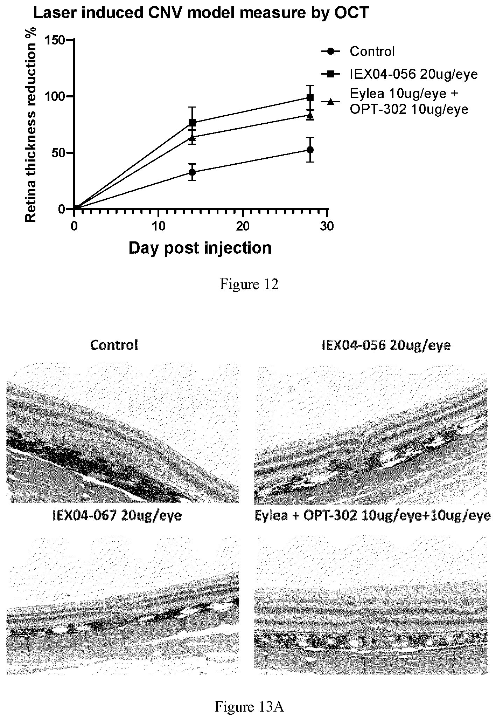

Images were taken by a microscope to calculate the number of lumens. The test results are as shown in Fig. 8A to Fig. 8B. FIG. 12. Fig. 8A shows lumen formation images; and Fig. 8B shows lumen formation statistics. The anti-VEGFA and anti-VEGFC bispecific antibodies were able to completely inhibit VEGFA + VEGFC-induced HUVEC cell lumen formation.

**Example 11: Assay of Inhibiting A375 Tumor Neovascularization by Anti-VEGFA/VEGFC Antibody**

The overexpression of VEGFA and VEGFC in tumor cells could induce neovascularization formation and promote tumor growth in vivo.

In this example, the anti-neovascularization and anti-tumor effects of the anti-VEGFA/VEGFC antibody of the present application were determined in nude mice by inoculating A375 human malignant melanoma cells at 3*$10^6$ cells per mouse.

Human nude mice:

Female BALB/c background nude mice graded as SPF were purchased from Charles River, Beijing. The mice were domesticated for 7 days upon arrival, and the study was subsequently initiated.

Cells:

Human A375 cells were purchased from ATCC (CAT#: CRL-1619) and were routinely passaged and cultured in strict accordance with the instructions for subsequent assays in vivo. The cells were collected by centrifugation, resuspended in sterile PBS and adjusted to a cell density of $1.5 \times 10^7$ cells/mL. On Day 0, 0.2 mL of cell suspension was subcutaneously inoculated into the axilla of human mice to establish A375 tumor-bearing mouse models.

Administration:

The mice were randomly grouped (6 mice in each group). The tumor volume of each mouse was detected 7 and 21 days after tumor cell inoculation. The dosage and mode of administration were as shown in Table 3, and PBS (purchased from Gibco) was taken as a negative control. The doses were administered on Days 1, 3, 5, 7, 9, 11, 13, 15, 17, and 19 after inoculation, and the tumor volume and body weight of the mice were monitored twice a week. The body weight and tumor volume were measured before each administration, and the relative tumor inhibition rate (TGI%) was calculated on Day 21 after inoculation with the following formula: TGI% = 100% * (the tumor volume of control group - the tumor volume of treatment group)/(the tumor volume of control group - the tumor volume of control group before administration).

Tumor volume measurement: the maximum length axis (L) and the maximum width axis (W) of each tumor were measured using a vernier caliper, and the tumor volume was calculated according to the following formula: $V = L \times W^2/2$. The body weight was measurement using an electronic balance.

Table 3. Experimental design

| Group | Administrated dose | Number of administration | Administration mode |
|---|---|---|---|
| PBS | / | Q2D×10 | Intraperitoneal injection |
| IEX04-056 | 1 mg/kg | Q2D×10 | Intraperitoneal injection |
| IEX04-056 | 5 mg/kg | Q2D×10 | Intraperitoneal injection |
| IEX04-056 | 25 mg/kg | Q2D×10 | Intraperitoneal injection |

The results of tumor inhibition rate are shown in Fig. 9A, Fig. 9B and Table 4: on Day 21 after inoculation, single IEX04-056 showed a tumor inhibition effect at different doses. On Day 21 after inoculation, the tumor inhibition rate was 54% at a dose of 1 mg/kg, 71% at a dose of 5 mg/kg, and 77% at a dose of 25 mg/kg. Therefore, the anti-VEGFA/VEGFC bispecific binding protein of the present application showed a significant tumor inhibitory effect.

Table 4. Tumor inhibition rate on Day 21

| Group | Average tumor volume (mm$^3$) | TGI(%) |
|---|---|---|
| PBS | 362 | / |
| IEX04-056 1mg/kg | 163 | 54% |
| IEX04-056 5mg/kg | 106 | 71% |
| IEX04-056 25mg/kg | 84 | 77% |

Tumor tissues on Days 7 and 21 after inoculation were subjected to sectioning and CD31 (vascular endothelial marker) staining, showing microangiogenesis in tumor tissues. The paraffin blocks embedded in an OCT embedding agent were placed in a frozen sectioning machine, and cut into sections with the thickness of 6 μm.

1. The sections were left at room temperature for 30 min, followed by fixation with pre-cooled acetone at 4°C and air drying at room temperature.
2. The sections were soaked and washed PBST three times, once for 5 min (PBST. 1 × PBS + 0.05% Tween 20).
3. An immunohistochemistry pen was used to draw circles, followed by sealing with 10% goat serum (diluted in PBST) dropwise added at 100 μL per tissue at room temperature.
4. 100 μL of primary antibody PE-anti-mouse CD31 was directly dropwise added and incubated overnight at 4°C.
5. On the next day, the tissues were soaked and washed with PBST three times, once for 5 min.
6. DAPI staining of cell nuclei was carried out by dropwise adding 100 μl to each tissue, which was then incubated in the dark for 5 min.
7. The DAPI staining solution was discarded, the tissues were soaked and washed with PBST three times, once for 5 min.

The sections were mounted with an anti-fluorescence quenching mountant (prolong diamond antifade mountant, Invitrogen). Subsequently, the mounted sections were scanned with a full-automatic quantitative analysis scanner. The results were shown in Fig. 9C, indicating a significant decrease in neovascularization in A375 tumors after IEX04-056 treatment.

**Example 12: Efficacy Assay for Laser-induced Choroidal Neovascularization**

In this assay, the anti-neovascularization effect of the anti-VEGFA/VEGFC bispecific antibody of the present application was tested using the cynomolgus monkey model of laser-induced choroidal neovascularization. Species: cynomolgus monkeys; grade: general grade; age: 2.5-5 years old; body weight: 2.45 kg to 5.55 kg, with the mean of 4.02 kg; body weight during modeling: 3.35 kg to 4.35 kg.

In this assay, laser photocoagulation was carried out around the fundus macular center of each rhesus monkey fundus to induce choroidal vascular neovascularization in the fundus, and to establish an animal model similar to human choroidal neovascularization. Fundus fluorescein angiography was carried out before and 20 days after photocoagulation to determine the modeling status. 12 successfully modeled rhesus monkeys (half male and half female) were divided into three groups: a model control group, an IEX04-056 group, and an Eylea + OPT-302 group, with four monkeys in each group. 21 days after photocoagulation, the doses were administered according to the dosages in Table 5 below. IEX04-056 or Eylea+OPT-302 was given by intravitreal injection in both eyes, and an equal volume of 0.9% sodium chloride injection was given to the model control group. The animals in each group were subjected to fundus color photography, fundus fluorescein angiography , and optical coherence tomography examination on Days 7, 14, 21, and 28 after administration, respectively, to observe the inhibition of choroidal neovascularization by the test article. After euthanasia on Day 29 after administration, both eyes were harvested for HE staining and histological examination.

Table 5. Experimental design

| Group | Dose | Dosage volume | Concentration | Administration mode |
|---|---|---|---|---|
| Control | / | / | / | / |
| IEX04-056 | 20 μg/eye | 50 μL/eye | 0.4 mg/mL | Intravitreal administration |
| Eylea+OPT-302 | 10 μg/eye +10 μg/eye | 50 μL/eye | 0.4 mg/mL | Intravitreal administration |

Fundus color photography and fundus fluorescein angiography

Evaluation indicators:

(1) Grading of fluorescent spots

The grading criteria for fluorescent spots taken by fluorescein angiography after modeling:

Grade 1: the fluorescent spot did not show hyperfluorescence;
Grade 2: the fluorescent spot showed hyperfluorescent but without fluorescein leakage;
Grade 3: the fluorescent spot showed hyperfluorescent and slight fluorescein leakage that did not exceed the edge of the spot;
Grade 4: the fluorescent spot showed hyperfluorescent and significant fluorescein leakage that exceeded the edge of the spot;
The fluorescent spots of Grades 1-4 were counted, and the grade of the fundus laser spot should be recorded for each examination.

(2) Improvement rate of fluorescein leakage area

Improvement rate of fluorescein leakage area (%) = (fluorescein leakage area before administration - fluorescein leakage area after administration)/fluorescein leakage area before administration * 100%.

(3) Reduced fluorescein leakage area

Reduced fluorescein leakage area = fluorescein leakage area before administration - fluorescein leakage area after administration

Optical coherence tomography (OCT)

Evaluation indicators:

(1) Improvement rate of fundus retinal thickening

Improvement rate of fundus retinal fickening (%)

$$= \frac{\text{retinal thickness before administration} - \text{retinal thickness after administration}}{\text{retinal thickness before administration} - \text{retinal thickness before modeling}}$$

$$\times 100\%$$

(2) Reduced fundus retinal thickness

Reduced fundus retinal thickness reduction = retinal thickness before administration - retinal thickness after administration

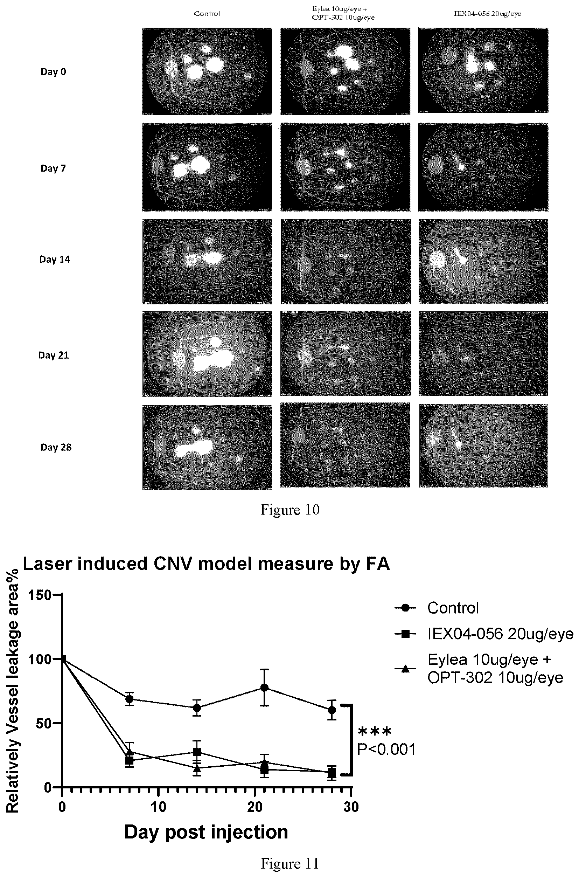

The results of fundus color photography and fluorescein angiography were shown in Fig. 10 to Fig. 11, showing a significant anti-neovascularization effect of the antibody of the present application 28 days after administration, which proved that the antibody of the present application had a significant inhibitory effect (P < 0.001) on laser-induced fundus neovascularization, and also had the function of protecting the integrity of blood vessels. The OCT results were shown in Fig. 12, indicating that the antibody of the present application significantly inhibited retinal thickening (P<0.05) 14 to 28 days after administration, which proved that the antibody of the present application had the function of inhibiting retinal edema and thickening caused by neovascularization.

Histopathological examination

29 days after administration, the rhesus monkeys were anesthetized with sodium pentobarbital (intravenous injection at about 30 mg/kg; the dose could be adjusted according to the health status of the animals) based on body weight, and euthanized by bleeding from the abdominal aorta or femoral artery for gross observation, and the eyes were removed bilaterally.

Both eyes of some animals were fixed with a modified Davidson's fixative and sectioned after paraffin embedding, and the laser modeling area was selected for routine HE staining and other histopathological examinations.

The antibody of the present application could better ameliorate pathological changes such as edema, hyperplasia, and fibrosis at the site of laser injury in pathological sections with respect to the combination of the anti-VEGFA molecules Eylea and the anti-VEGFC molecules OPT-032, showing better morphological improvement of the retina (Fig. 13A and Fig. 13B).

Next, the effects of different pHs and different excipients (e.g., sorbitol, sucrose, trehalose, arginine, sodium chloride, polysorbate 20, and polysorbate 80) on the stability of proteins were investigated by high-temperature experiments.

**Example 13:** Preliminary Screening of Formulations for Low-concentration Preparations - Pre-formulation Studies

A buffer containing 1.55 mg/mL histidine and 50.00 mg/mL sorbitol was prepared, with the pH adjusted to 5.0, 5.5, 6.0, 6.5, and 7.0 with a dilute hydrochloric acid solution, respectively, the anti-VEGFA/VEGFC bispecific binding proteins prepared as described above were ultrafiltered and replaced into buffers with different pH values, and the protein content was adjusted to about 120 mg/mL; polysorbate 80 was added to achieve its final concentration of 0.2 mg/mL; and the mixtures were filtered and dispensed into vials, which were than stoppered and capped. The stability of the above samples was investigated at 40°C $\pm$ 2°C, with the specific protocol shown in Table 6.

Table 6. Pre-formulation experimental protocol

| Experimental conditions | Sampling point | | | | Test item |
|---|---|---|---|---|---|
| | Week 0 | Week 1 | Week 2 | Week 4 | |
| 40°C±2°C | √ | N/A | √ | N/A | Appearance, visible foreign matter, protein content, pH, purity, charge variant and activity |
| Note: (1) √ indicates a site for sampling. (2) N/A indicates absence of the identified time point. | | | | | |

Based on the knowledge of the samples to be tested and the accuracy of the instruments and methods, the criteria for determining unchanged quality of the samples as compared to the detected values and initial values. The criteria for determining unchanged quality of products were not included, since the standard methods for testing purity, charge isomers and activity had not yet been established during the pre-formulation study. The criteria were specified in Table 7.

Table 7. Criteria for determining unchanged quality

| Test item | Criteria for determining no change |
|---|---|
| Appearance (observation method) | Clear to light opalescent, colorless to light yellow liquid, no foreign matter |
| Visible foreign matters (visible foreign matter inspection method)[1] | No foreign matter |
| Protein content (UV method) | Change rate ≤ 10% |
| pH value (pH value determination method) | Change value ≤ 0.3 |
| Note: [1] This criterion is only applicable to pre-formulation studies. | |

Experimental results

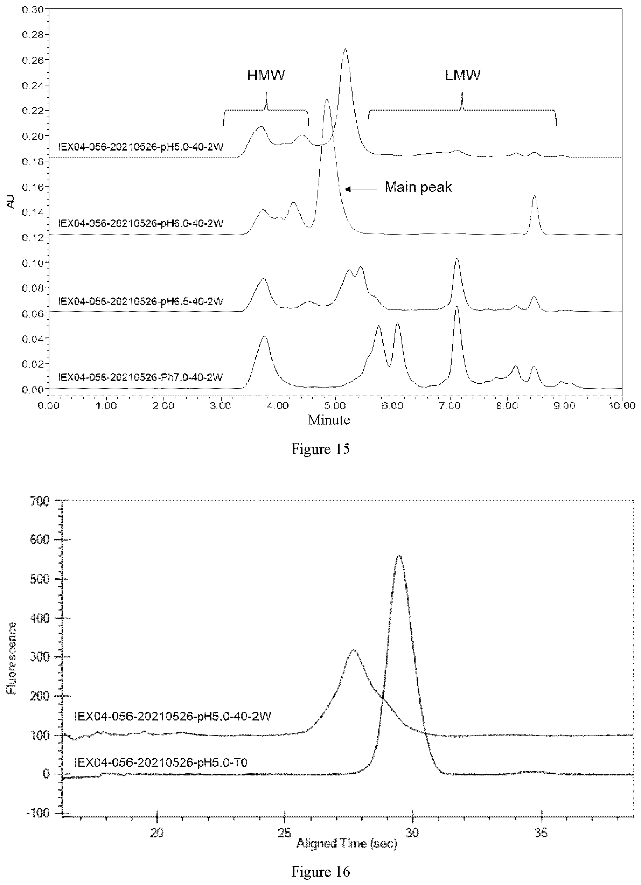

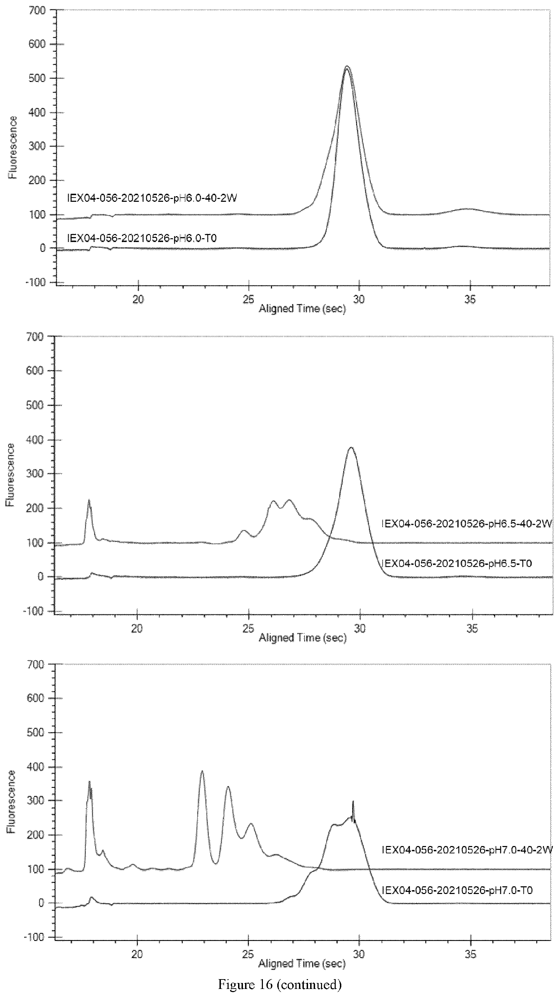

The results of the pre-formulation study showed that, when kept at 40°C ± 2°C for 2 weeks, the samples at pH 5.0 became turbid in appearance, and the samples under other pH conditions were qualified both in appearance and visible foreign matter. The peak shapes of purity (SEC-HPLC and non-reduced CE-SDS) of the samples under different pH conditions showed significant difference, as shown in Fig. 15 and Fig. 16. The samples at pH 6.0 exhibited better purity than the samples under other pH conditions. Subsequent formulation determination assays were carried out at pH 6.2.

**Example 14:** Screening of Formulation for Low-concentration Preparation - Formulation Determination Assay In this assay, the effect of excipients (histidine, sodium dihydrogen phosphate, sorbitol, sucrose, trehalose, arginine, sodium chloride, polysorbate 20 and polysorbate 80) on the stability of proteins was investigated, and the detailed formulation information was shown in Table 8.

The buffers for respective formulations were prepared according to Table 8, and the proteins were ultrafiltered and replaced into their respective formulation solutions. After the replacement was completed, the protein contents of Formulations 1 to 4 (F1 to F4) were adjusted to 50 mg/mL; polysorbate 80 or polysorbate 20 was added to its final concentration of 0.3 mg/mL; and the samples were filtered and dispensed into vials, which were then stoppered and capped. The above samples were investigated for stability at 40°C ± 2°C.

Table 8. Information of candidate formulations

| No. | Formulation information |
|---|---|
| Formulation 1 | 2.68 mg/mL sodium dihydrogen phosphate, 50.00 mg/mL sucrose, 2.34 mg/mL sodium chloride, and 0.3 mg/mL polysorbate 20, pH 6.2 |
| Formulation 2 | 1.55 mg/mL histidine, 50.00 mg/mL sorbitol, and 0.3 mg/mL polysorbate 80, pH 6.2 |
| Formulation 3 | 1.55 mg/mL histidine, 60.00 mg/mL sucrose, 13.94 mg/mL arginine, and 0.3 mg/mL polysorbate 80, pH 6.2 |
| Formulation 4 | 1.55 mg/mL histidine, 80.00 mg/mL trehalose, and 0.3 mg/mL polysorbate 80, pH 6.2 |
| Note: the pH was adjusted with the dilute hydrochloric acid solution. | |

The stability investigation protocol was shown in Table 9, the criteria were shown in Table 10, and the investigation results were shown in Table 11.

Table 9. Stability investigation protocol

| Formulation | Experimental protocol and sampling point | Test item |
|---|---|---|
| Formulation 1 to Formulation 4 | The samples were kept at 40°C ± 2°C and sampled on Day 0, Week 1, Week 2 and Week 4. | Appearance, visible foreign matter, pH, protein content, purity. |
| Note: All samples taken at the above time points were first placed in a refrigerator at -70°C to be cryopreserved for testing, and then thawed and sent for testing as required. | | |

Table 10. Criteria for determining unchanged quality

| Test item | Criteria for no change |
|---|---|
| Appearance (observation method) | Clear to light opalescent, colorless to light yellow liquid, no foreign matter |
| Visible foreign matters (visible foreign matter test method) * | No foreign matter |
| Protein content (UV method) | Change rate ≤ 10% |
| pH value | Change value ≤ 0.3 |
| Purity (SEC-HPLC method) | Major peak change value ≤ 1.0% |
| Purity (non-reduced CE-SDS method) | Major peak change value ≤ 2.0% |

Table 11. Investigation results

| Test item | | Time | Test results | | | |
|---|---|---|---|---|---|---|
| | | | Formu lation 1 | Formula tion 2 | Formul ation 3 | Formul ation 4 |
| Appearance (observation method) | | Week 0 | Accep table | Accepta ble | Accept able | Accept able |
| | | Week 1 | Accep table | Accepta ble | Accept able | Accept able |
| | | Week 2 | Accep table | Accepta ble | Accept able | Accept able |
| | | Week 4 | Accep table | Accepta ble | Accept able | Accept able |
| Visible foreign matters (visible foreign matter inspection method) | | Week 0 | Accep table | Accepta ble | Accept able | Accept able |
| | | Week 1 | Accep table | Accepta ble | Accept able | Accept able |
| | | Week 2 | Accep table | Accepta ble | Accept able | Accept able |
| | | Week 4 | Accep table | Accepta ble | Accept able | Accept able |
| pH (pH determination method) | | Week 0 | 6.2 | 6.2 | 6.2 | 6.2 |
| | | Week 1 | 6.2 | 6.2 | 6.2 | 6.2 |
| | | Week 2 | 6.2 | 6.2 | 6.2 | 6.2 |
| | | Week 4 | N/A | N/A | N/A | N/A |
| Protein content (UV method, mg/mL) | | Week 0 | 50.1 | 49.2 | 51.3 | 49.4 |
| | | Week 1 | 50.3 | 47.5 | 51.2 | 48.5 |
| | | Week 2 | 50.3 | 48.6 | 52.0 | 49.0 |
| | | Week 4 | 51.3 | 50.2 | 52.4 | 49.1 |
| Purity (SEC-HPLC method, %) | Major peak | Week 0 | 93.7 | 93.7 | 94.2 | 93.7 |
| | | Week 1 | 73.9 | 83.7 | 79.8 | 85.9 |
| | | Week 2 | 68.2 | 79.0 | 69.9 | 81.5 |
| | | Week 4 | 69.9 | 78.6 | 58.8 | 73.5 |

(continued)

| Test item | | Time | Test results | | | |
|---|---|---|---|---|---|---|
| | | | Formu lation 1 | Formula tion 2 | Formul ation 3 | Formul ation 4 |
| | Polymer | Week 0 | 6.3 | 5.8 | 5.8 | 6.3 |
| | | Week 1 | 23.8 | 11.6 | 18.5 | 13.6 |
| | | Week 2 | 27.9 | 14.4 | 27.4 | 17.6 |
| | | Week 4 | 26.0 | 15.2 | 37.3 | 25.7 |
| Purity (non-reduced CE-SDS method, %) | Major peak | Week 0 | 97.5 | 97.2 | 97.7 | 97.5 |
| | | Week 1 | 91.3 | 91.1 | 93.7 | 96.1 |
| | | Week 2 | N/A | N/A | N/A | N/A |
| | Fragment | Week 0 | 0.9 | 1.3 | 0.8 | 0.9 |
| | | Week 1 | 5.5 | 7.2 | 3.7 | 1.3 |
| | | Week 2 | N/A | N/A | N/A | N/A |
| | Polymer | Week 0 | 1.6 | 1.5 | 1.5 | 1.5 |
| | | Week 1 | 3.3 | 1.6 | 2.7 | 2.6 |
| | | Week 2 | N/A | N/A | N/A | N/A |
| Note: N/A indicates undetected. | | | | | | |

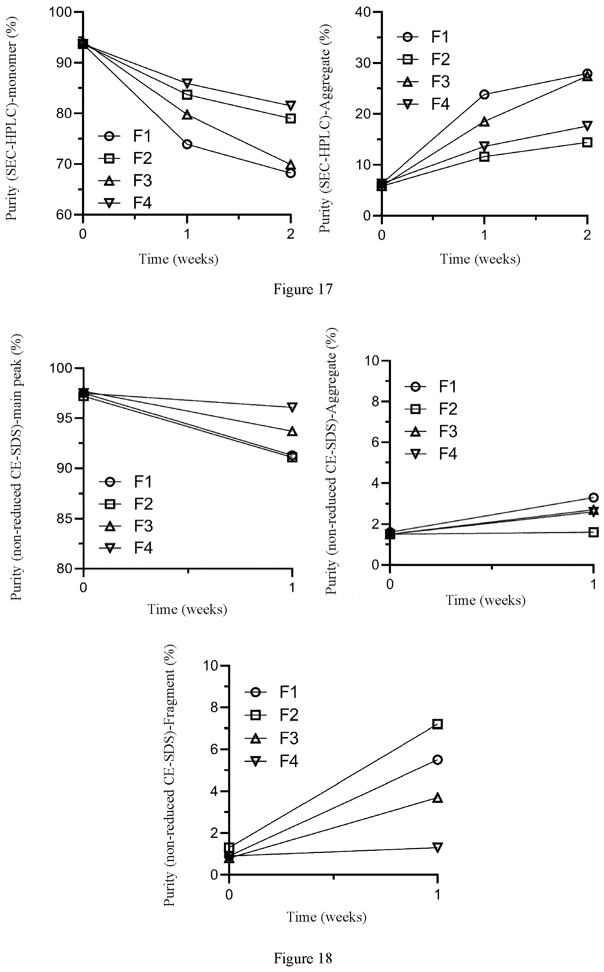

The results in Table 11 showed that, when the samples were kept at at 40°C $\pm$ 2°C for 2 weeks, the samples of different formulations were qualified in appearance and visible foreign matter; the protein content and pH value did not show significant changes; the purity of all the samples of all the formulations (SEC-HPLC and non-reduced CE-SDS methods) changed significantly, and the purity of the samples of Formulation 4 (SEC-HPLC and non-reduced CE-SDS methods) showed the minimum decrease. The specific trend charts were shown in Fig. 17 and Fig. 18.

In order to avoid adjusting pH with hydrochloric acid during production, the buffering system was adjusted to a mixed solution of histidine and L-histidine hydrochloride, and meanwhile, because of the low osmotic pressure of the preparation of Formulation 4 (262 mOsmol/kg), the concentration of trehalose was increased to 90 mg/mL (at which the osmotic pressure of the preparation was 323 mOsmol/kg) to achieve isotonic osmotic pressure for the buffer of the preparation. That is, the final formulation of the preparation included: 50.0 mg/mL recombinant bispecific binding proteins against vascular endothelial growth factor A (VEGFA) and vascular endothelial growth factor C (VEGFC), 0.90 mg/mL histidine, 0.90 mg/mL L-histidine hydrochloride, 90.00 mg/mL trehalose and 0.3 mg/mL polysorbate 80, with the pH of 6.2.

By combining the results of the above assays, it was determined that the final formulation of the preparation included: 50.0 mg/mL recombinant bispecific binding proteins against vascular endothelial growth factor A (VEGFA) and vascular endothelial growth factor C (VEGFC), 0.90 mg/mL histidine, 0.90 mg/mL L-histidine hydrochloride, 90.00 mg/mL trehalose and 0.3 mg/mL polysorbate 80, with the pH of 6.2. With the use of this formulation of preparation, the anti-VEGFA/VEGFC bispecific binding proteins of the present application can exhibit good stability and thus exhibit the desired safety and good efficacy clinically.

Those of ordinary skill in the art to which the present application belongs should understand that the discussion of any of the above embodiments is merely for an exemplary purpose, and is not intended to imply that the protection scope of the present disclosure is limited to these embodiments. Under the concept of the present disclosure, the above embodiments or the technical features in different embodiments can also be combined; and the steps can be implemented in any order. Moreover, there are many other variations in different aspects of the embodiments of the present disclosure as described above, and these variations are not provided in detail for the sake of brevity. The embodiments of the present disclosure are intended to cover all such substitutions, modifications and variations that fall within the broad scope of the appended claims. Therefore, any omission, modification, equivalent substitution, improvement and the like made within the spirit and principle of the embodiments of the present disclosure shall be construed as being included in the protection scope of the present disclosure.

For the purposes of description and disclosure, all patents, patent applications, and other publications are hereby expressly incorporated herein by reference. These publications are provided solely because their disclosures precede the filing date of the present application. All statements regarding the dates of these documents or representations of their contents are based on information available to the applicant and do not constitute any acknowledgment of the correctness of the dates or contents of these documents. Moreover, in any country, any reference to these publications herein does not constitute the recognition that these publications have become part of the common knowledge in the art.

**Claims**

1.  A pharmaceutical preparation, comprising: (1) an anti-VEGFA/VEGFC bispecific binding protein, (2) a buffering agent, (3) a stabilizer, and (4) a surfactant, wherein said pharmaceutical preparation has a pH of 5.5 to 7.0.

2.  The pharmaceutical preparation according to claim 1, wherein said bispecific binding protein has a concentration of less than 80 mg/mL and greater than 0;
    or said bispecific binding protein has a concentration of 20 mg/mL to 80 mg/mL, 50 mg/mL to 80 mg/mL, 40 mg/mL to 60 mg/mL, or 45 mg/mL to 65 mg/mL.

3.  The pharmaceutical preparation according to claim 1 or 2, wherein said buffering agent is a phosphoric acid or phosphate buffering system, or a histidine buffering system;

    preferably, said histidine buffering system comprises histidine, or histidine and L-histidine hydrochloride;
    more preferably, said histidine buffering system comprises 1.0 mg/mL to 2.0 mg/mL, or 1.5 mg/mL to 1.6 mg/mL histidine; or said histidine buffering system comprises: 0.80 mg/mL to 1.5 mg/mL, 0.80 mg/mL to 1.0 mg/mL, 0.80 mg/mL to 0.95 mg/mL, 0.85 mg/mL to 1.0 mg/mL, or 0.85 mg/mL to 0.95 mg/mL histidine, and 0.80 mg/mL to 1.5 mg/mL, 0.80 mg/mL to 1.0 mg/mL, 0.80 mg/mL to 0.95 mg/mL, 0.85 mg/mL to 1.0 mg/mL, or 0.85 mg/mL to 0.95 mg/mL L-histidine hydrochloride.

4.  The pharmaceutical preparation according to any one of claims 1 to 3, wherein said stabilizer is a sugar, sugar alcohol or basic amino acid enabling stable presence of a protein;

    preferably, said sugar is trehalose or sucrose;
    said sugar alcohol is sorbitol or mannitol; or
    said basic amino acid is arginine or lysine.

5.  The pharmaceutical preparation according to claim 4, wherein said sugar has a concentration of 50 mg/mL to 100 mg/mL, 80 mg/mL to 100 mg/mL, 85 mg/mL to 100 mg/mL, 80 mg/mL to 95 mg/mL, 85 mg/mL to 95 mg/mL, 80 mg/mL to 90 mg/mL, or 85 mg/mL to 90 mg/mL;

    said sugar alcohol has a concentration of 30 mg/mL to 80 mg/mL, 40 mg/mL to 60 mg/mL, 45 mg/mL to 55 mg/mL, or 50 mg/mL to 55 mg/mL; or
    said basic amino acid has a concentration of 5 mg/mL to 30 mg/mL, 10 mg/mL to 20 mg/mL, 10 mg/mL to 15 mg/mL, 11 mg/mL to 15 mg/mL, 12 mg/mL to 15 mg/mL, or 13 mg/mL to 15 mg/mL.

6.  The pharmaceutical preparation according to claim 4 or 5, wherein said sugar is trehalose with a concentration of 80 mg/mL to 100 mg/mL, 85 mg/mL to 100 mg/mL, 85 mg/mL to 95 mg/mL, 80 mg/mL to 90 mg/mL, or 85 mg/mL to 90 mg/mL.

7.  The pharmaceutical preparation according to any one of claims 1 to 6, wherein said surfactant is a non-ionic surfactant;

    preferably, said surfactant is polysorbate;
    more preferably, said surfactant is polysorbate 20, or polysorbate 80;
    or preferably, said surfactant is polysorbate with a concentration of 0.1 mg/mL to 0.5 mg/mL, 0.25 mg/mL to 0.45 mg/mL, 0.2 mg/mL to 0.4 mg/mL, 0.25 mg/mL to 0.4 mg/mL, or 0.3 mg/mL to 0.4 mg/mL; or
    more preferably, said surfactant is polysorbate 80 with a concentration of 0.2 mg/mL to 0.4 mg/mL, 0.25 mg/mL to 0.4 mg/mL, or 0.3 mg/mL to 0.4 mg/mL.

8.  The pharmaceutical preparation according to any one of claims 1 to 7, wherein said pharmaceutical preparation is a liquid injection or lyophilized powder; and
    preferably, said pharmaceutical preparation has a pH of 5.8 to 6.5, 6.0 to 6.5, or 6.0 to 6.2.

9.  The pharmaceutical preparation according to any one of claims 1 to 8, wherein said bispecific binding protein comprises:

(i) a first antigen binding component specifically binding to human VEGFC, wherein said first antigen binding component comprises an anti-VEGFC single-domain antibody or an anti-VEGFC VHH domain, and said single-domain antibody or said VHH domain comprises: CDR1 having an amino acid as set forth in SEQ ID NO: 1, CDR2 having an amino acid sequence as set forth in SEQ ID NO: 2, and CDR3 having an amino acid sequence as set forth in SEQ ID NO: 3; and
(ii) a second antigen binding component specifically binding to human VEGFA.

10. The pharmaceutical preparation according to claim 9, wherein said first antigen binding component is a fusion protein comprising said anti-VEGFC single-domain antibody, or a VHH domain thereof, and an Fc moiety; preferably, said fusion protein comprises said single-domain antibody, or the VHH domain thereof, and said Fc moiety at a C-terminus of said single-domain antibody or the VHH domain thereof; more preferably, said VHH domain comprises : (i) an amino acid as set forth in SEQ ID NO: 4, (ii) an amino acid sequence having at least 80%, 85%, 90%, 91%, 92%, 93%, 94%, 95%, 96%, 97%, 98%, 99% or more identity to the amino acid sequence in (i), or (iii) an amino acid sequence having at least 1 to 30, or 1 to 20, or 1 to 15, or 1 to 10, or 1 to 5 amino acid changes with respect to the amino acid sequence in (i); further preferably, said VHH domain comprises an amino acid sequence as set forth in SEQ ID NO: 4 or consists of an amino acid sequence as set forth in SEQ ID NO: 4;
or
said second antigen binding component is a fusion protein comprising an Fc moiety and a VEGFA binding domain; preferably, said VEGFA binding domain comprises or consists of: an amino acid sequence as set forth in SEQ ID NO: 6, or an amino acid sequence having at least 90%, 91%, 92%, 93%, 94%, 95%, 96%, 97%, 98%, 99% or more identity to the amino acid sequence as set forth in SEQ ID NO: 6; more preferably, said VEGFA binding domain comprises an amino acid sequence as set forth in SEQ ID NO: 6 or consists of an amino acid sequence as set forth in SEQ ID NO: 6.

11. The pharmaceutical preparation according to claim 10, wherein said Fc moiety is a human IgG1 Fc region, which comprises or consists of: an amino acid sequence as set forth in SEQ ID NO: 7, or an amino acid sequence having at least 90%, 91%, 92%, 93%, 94%, 95%, 96%, 97%, 98%, 99% or more identity to the amino acid sequence as set forth in SEQ ID NO: 7.

12. The pharmaceutical preparation according to any one of claims 9 to 11, wherein said second antigen binding component comprises or consists of: an amino acid sequence as set forth in SEQ ID NO: 5, or an amino acid sequence having at least 90%, 91%, 92%, 93%, 94%, 95%, 96%, 97%, 98%, 99% or more identity to the amino acid sequence as set forth in SEQ ID NO: 5.

13. The pharmaceutical preparation according to any one of claims 9 to 12, wherein said first antigen binding component is linked or fused at a C-terminus or an N-terminus of said second antigen binding component;
or, said bispecific binding protein comprises a linker linking said first antigen binding component to said second antigen binding component, and said linker has an amino acid sequence of (G4S)n or G(G4S)n, with n=1, 2, 3, 4 or 5.

14. The pharmaceutical preparation according to any one of claims 1 to 13, wherein said bispecific binding protein comprises a first polypeptide chain and a second polypeptide chain, and said first polypeptide chain and said second polypeptide chain are identical and each comprise an amino acid sequence as set forth in SEQ ID NO: 8.

15. The pharmaceutical preparation according to any one of claims 1 to 14, wherein said pharmaceutical preparation comprises: 40 mg/mL to 80 mg/mL said anti-VEGFA/VEGFC bispecific binding protein, 0.80 mg/mL to 1.0 mg/mL histidine, 0.80 mg/mL to 1.0 mg/mL L-histidine hydrochloride, 80 mg/mL to 100 mg/mL sugar, and 0.2 mg/mL to 0.5 mg/mL polysorbate, said pharmaceutical preparation having a pH of 6.0 to 6.5;

or, said pharmaceutical preparation comprises: 40 mg/mL to 80 mg/mL said anti-VEGFA/VEGFC bispecific binding protein, 0.80 mg/mL to 1.0 mg/mL histidine, 0.80 mg/mL to 1.0 mg/mL L-histidine hydrochloride, 80 mg/mL to 100 mg/mL trehalose, and 0.2 mg/mL to 0.5 mg/mL polysorbate 80, said pharmaceutical preparation having a pH of 6.0 to 6.5;
or, said pharmaceutical preparation comprises: 40 mg/mL to 60 mg/mL said anti-VEGFA/VEGFC bispecific binding protein, 0.85 mg/mL to 0.95 mg/mL histidine, 0.85 mg/mL to 0.95 mg/mL L-histidine hydrochloride, 85 mg/mL to 100 mg/mL trehalose, and 0.2 mg/mL to 0.4 mg/mL polysorbate 80, said pharmaceutical preparation having a pH of 6.0 to 6.2;
or, said pharmaceutical preparation comprises: 50 mg/mL said anti-VEGFA/VEGFC bispecific binding protein, 0.90 mg/mL histidine, 0.90 mg/mL L-histidine hydrochloride, 90.00 mg/mL trehalose, and 0.3 mg/mL polysorbate 80, said pharmaceutical preparation having a pH of 6.2.

16. The pharmaceutical preparation according to any one of claims 1 to 15, wherein said pharmaceutical preparation has an osmotic pressure of 280 mOsmol/kg or more, 300 mOsmol/kg or more, or 300 mOsmol/kg to 350 mOsmol/kg.

17. The pharmaceutical preparation according to any one of claims 1 to 16, wherein said pharmaceutical preparation keeps stable at 30°C to 50°C for more than 2 or 4 weeks in a form of liquid or lyophilized powder; and
preferably, said pharmaceutical preparation, when stored at 30°C to 50°C for more than 2 weeks in a form of liquid, maintains a purity of more than 55%, more than 65%, more than 70%, more than 75%, or more than 80% as determined by an SEC-HPLC method.

18. The pharmaceutical preparation according to any one of claims 1 to 17 for use in treatment or diagnosis of neovascularization-related diseases, wherein

preferably, said diseases comprise solid tumors or ophthalmic diseases; and
more preferably, said diseases comprise melanoma, age-related macular degeneration, diabetic retinopathy, retinal vascular occlusion or corneal neovascularization.

19. A kit, comprising said pharmaceutical preparation of any one of claims 1 to 17.

20. The kit according to claim 19, wherein said kit further comprises: one or more additional activators, which are administrated in combination with said pharmaceutical preparation;

a device for administration to a subject; and/or
an instruction for use, a marker or a coupling reagent, and/or a pharmaceutical carrier;
preferably, said activator can be any one of: a chemotherapeutic agent, a radiotherapeutic agents, an anti-neovascularization drug, an immunosuppressive drug, an anti-fibrotic drug, a neuroprotective agent, and drugs with tumor-inhibiting effects; or preferably, said device for administration to a subject is a syringe, an inhalation device, an implantation device, or an infusion device.

Figure 1

**HEK 293 KDR reporter assay (VEGFC)**

| | IgG1 | am63G12.1-5G8-18B9 | Blank |
|---|---|---|---|
| IC50 | ~ 1.714 | ~ 4.457 | |

Figure 2

**anti-VEGF-C blocks FLT4-Ba/F3 proliferation**

| | hIgG1 | am63G12-5G8-18B9 |
|---|---|---|
| IC50 | 0.2535 | 0.7917 |

Figure 3

**HEK293 KDR reporter assay (VEGFA)**

| | IgG1 | IBI304 | Faricimab | IEX04-056 |
|---|---|---|---|---|
| IC50 | ~ 9.995 | 0.6115 | 1.128 | 0.6020 |

Figure 4

**HEK293 KDR reporter assay (VEGFC)**

| | IgG1 | OPT-302 | IEX04-056 |
|---|---|---|---|
| IC50 | ~ 0.05235 | 0.2407 | 0.4720 |

Figure 5

**Ba/F3-FLT4 proliferation assay (VEGFC)**

| | hIgG1 | OPT-302 | IEX04-056 |
|---|---|---|---|
| IC50 | ~ 2.572e-006 | 0.1681 | 0.2625 |

Figure 6

Figure 7

| | Blank | VEGF A | VEGF C |
|---|---|---|---|

| VEGF A + VEGF C | IBI304 | OPT - 302 |
|---|---|---|

| IBI304 + OPT - 302 | IEX04-056 | IEX04-067 |
|---|---|---|

Figure 8A

Figure 8B

Figure 9A

Figure 9B

Figure 9C

Figure 10

Figure 11

Figure 12

Figure 13A

# H&E staining

Figure 13B

Figure 14

Figure 15

Figure 16

Figure 16 (continued)

Figure 17

Figure 18

## INTERNATIONAL SEARCH REPORT

| International application No. |
|---|
| **PCT/CN2024/077354** |

| A. | CLASSIFICATION OF SUBJECT MATTER |
|---|---|

A61K39/395(2006.01)i；　C07K16/22(2006.01)i；　A61P35/00(2006.01)i

According to International Patent Classification (IPC) or to both national classification and IPC

| B. | FIELDS SEARCHED |
|---|---|

Minimum documentation searched (classification system followed by classification symbols)

　　　IPC:A61K,C07K,A61P

Documentation searched other than minimum documentation to the extent that such documents are included in the fields searched

Electronic data base consulted during the international search (name of data base and, where practicable, search terms used)

　　　CJFD, CNTXT, ENTXTC, DWPI, VEN, CNKI, BING. NCBI, EBI, STN, 百度学术, BAIDU SCHOLAR: 申请人/发明人, 双特异性, 双抗, bispecific, 抗体, 抗原结合分子, antibod+, 表活, 表面活性剂, 甘露醇, 海藻糖, 碱性氨基酸, 精氨酸, 聚山梨醇酯, 聚山梨酯, 赖氨酸, 磷酸缓冲, 磷酸盐缓冲, 醛醇, 醛醣醇, 融合蛋白, 山梨醇, 山梨糖醇, 糖醇, 吐温, 血管内皮生长因子a, 血管内皮生长因子c, 盐酸组氨酸, 蔗糖, 组氨酸缓冲, 组氨酸盐酸盐, PBS, tween, VEGF, VEGFA, VEGFA165, VEGFC, pH 5.5~7.0, VEGF-C, VEGF-A

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | ZHANG, Dapeng et al. "Suppression of Tumor Growth and Metastasis by Simultaneously Blocking Vascular Endothelial Growth Factor (VEGF)–A and VEGF-C with a Receptor-Immunoglobulin Fusion Protein"<br>*Cancer Research*, Vol. 70,, No. 6, 15 March 2010 (2010-03-15), 2495-2503<br>　　abstract and figure 1 | 1-20 |
| Y | CN 112739323 A (REGENERON PHARMACEUTICALS, INC.) 30 April 2021 (2021-04-30)<br>　　claims 1-20 | 1-20 |
| PY | WO 2023016516 A1 (INNOVENT BIOLOGICS (SUZHOU) CO. LTD.) 16 February 2023 (2023-02-16)<br>　　claims 1-24 | 1-20 |
| PY | WO 2023016449 A1 (CHENGDU YUANPU BIOTECHNOLOGY CO., LTD.) 16 February 2023 (2023-02-16)<br>　　claims 1-11 and 16-20 | 1-20 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 May 2024** | **11 May 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/077354** |

**C.    DOCUMENTS CONSIDERED TO BE RELEVANT**

| Category* | Citation of document, with indication, where appropriate, of the relevant passages | Relevant to claim No. |
|---|---|---|
| A | JIMENEZ, X. et al. "A recombinant, fully human, bispecific antibody neutralizes the biological activities mediated by both vascular endothelial growth factor receptors 2 and 3" *Molecular Cancer Therapeutics*, Vol. 4, No. 3, 31 March 2005 (2005-03-31), 427-434 <br>    abstract | abstract |
| A | 黄崇青等 (HUANG, Chongqing et al.). "VEGF-A 和 VEGF-C双拮抗抑制 血管瘤内皮细胞增殖的实验研究 (Effect of VEGF-A and VEGF-C Co-inhibition on Proliferation of Hemangioma Endothelial Cells)" <br>*浙江医学 (Zhejiang Medical Journal)*, Vol. 43, No. 4, 31 December 2021 (2021-12-31), 364-372 <br>    abstract | 1-20 |

Form PCT/ISA/210 (second sheet) (July 2022)

**INTERNATIONAL SEARCH REPORT**

International application No.

**PCT/CN2024/077354**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2024/077354**

| Patent document cited in search report | | | Publication date (day/month/year) | Patent family member(s) | | | Publication date (day/month/year) |
|---|---|---|---|---|---|---|---|
| CN | 112739323 | A | 30 April 2021 | CO | 2020014427 | A2 | 21 December 2020 |
| | | | | BR | 112020022610 | A2 | 09 February 2021 |
| | | | | JOP | 20200275 | A1 | 02 November 2020 |
| | | | | CL | 2020002872 | A1 | 26 March 2021 |
| | | | | US | 2024058418 | A1 | 22 February 2024 |
| | | | | SG | 11202010684 | YA | 27 November 2020 |
| | | | | KR | 20210021299 | A | 25 February 2021 |
| | | | | JP | 2021523162 | A | 02 September 2021 |
| | | | | JP | 7235770 | B2 | 08 March 2023 |
| | | | | MA | 52570 | A | 17 March 2021 |
| | | | | JP | 2023071798 | A | 23 May 2023 |
| | | | | CL | 2021001957 | A1 | 08 April 2022 |
| | | | | MX | 2020011848 | A | 29 March 2021 |
| | | | | AU | 2019265005 | A1 | 17 December 2020 |
| | | | | WO | 2019217927 | A1 | 14 November 2019 |
| | | | | US | 2021353714 | A1 | 18 November 2021 |
| | | | | US | 2019343918 | A1 | 14 November 2019 |
| | | | | US | 11103552 | B2 | 31 August 2021 |
| | | | | EP | 3790532 | A1 | 17 March 2021 |
| | | | | PH | 12020551839 | A1 | 28 June 2021 |
| | | | | CA | 3099551 | A1 | 14 November 2019 |
| WO | 2023016516 | A1 | 16 February 2023 | AU | 2022327397 | A1 | 14 March 2024 |
| | | | | CA | 3229250 | A1 | 16 February 2023 |
| WO | 2023016449 | A1 | 16 February 2023 | AU | 2022325360 | A1 | 21 March 2024 |
| | | | | CA | 3228501 | A1 | 16 February 2023 |

Form PCT/ISA/210 (patent family annex) (July 2022)

## EP 4 663 202 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SINGLETON et al.** Dictionary of Microbiology and Molecular Biology. J. Wiley & Sons, 1994
- **SAMBROOK et al.** Molecular Cloning, A Laboratory Manual. Cold Springs Harbor Press, 1989
- Current Protocols in Molecular Biology or Current Protocols in Immunology. John Wiley & Sons, 2009
- **PERBAL**. *A Practical Guide to Molecular Cloning*, 1984
- **KABAT et al.** Sequences of Proteins of Immunological Interest. Department of Health and Human Services, National Institutes of Health, 1987
- **NORTH et al.** A New Clustering of Antibody CDR Loop Conformations. *Journal of Molecular Biology*, 2011, vol. 406, 228-256
- **KABAT et al.** Sequences of Proteins of Immunological Interest. Public Health Service, National Institutes of Health, 1991
- **ESTEP, P et al.** High throughput solution based measurement of antibody-antigen affinity and epitope binning. *MAbs*, 2013, vol. 5 (2), 270-8